# EUROPEAN PATENT SPECIFICATION

(11) **EP 1 492 527 B1**
(45) Date of publication and mention of the grant of the patent: **08.10.2008**
(21) Application number: 03719615.1
(22) Date of filing: 07.04.2003
(51) Int. Cl.: A61K 31/42, A61K 9/107, A61K 9/48, A61K 31/02

(54) **PROCESS FOR PREPARING A FINELY SELF-EMULSIFIABLE PHARMACEUTICAL COMPOSITION**
VERFAHREN ZUR HERSTELLUNG EINER FEIN SELBSTEMULGIERBAREN PHARMAZEUTISCHEN ZUSAMMENSETZUNG
PROCEDE POUR PREPARER UNE COMPOSITION PHARMACEUTIQUE FINEMENT AUTO-EMULSIFIABLE

(30) Priority: 09.04.2002 US 371200 P
(43) Date of publication of application: 05.01.2005
(73) Proprietor: Pharmacia Corporation, Peapack, NJ 07977 (US)
(72) Inventor: GAO,Ping, Portage, MI 49024 (US); HE, Xioarong, Kalamazoo, MI 49002 (US); BOLYARD, Keith, B., Otsego, MI 49078 (US)
(74) Representative: Wood, David John
(86) International application number: PCT/US2003/010526
(87) International publication number: WO 2003/086392

(56) References cited:
- WO-A-01/91750
- WO-A-02/05799
- WO-A-02/083177

## Description

### FIELD OF THE INVENTION

The present invention relates to processes for preparing orally deliverable finely self-emulsifiable pharmaceutical compositions that comprise a drug of low water solubility, more particularly to such compositions where the drug is in dissolved form.

### BACKGROUND OF THE INVENTION

Liquid dosage forms, for example solutions suitable for oral administration, have become an important method by which drugs are delivered to subjects, particularly where rapid onset of therapeutic effect is desired. As an alternative to directly imbibable liquid formulations of a drug, it is also known to encapsulate liquid formulations, for example in soft or hard gelatin capsules, to provide a discrete dosage form.

Unfortunately, many useful drugs have low solubility in water and, therefore, are difficult to formulate at convenient concentrations as solutions in an aqueous vehicle. Even when a suitable solvent is found as a vehicle for such a drug, there is often a tendency, particularly for a crystalline drug of low water solubility, to precipitate out of solution and/or crystallize when the drug comes in contact with water, for example in the aqueous environment of the gastrointestinal tract. Upon precipitation and/or crystallization, the drug can then agglomerate to form larger particles that further retard absorption. Such precipitation and/or crystallization, especially if accompanied by agglomeration, can offset or reduce the potential rapid onset benefits sought by formulating the drug as a solution.

Attempts have been made to facilitate gastrointestinal absorption of poorly water-soluble drugs from solution formulations, by adding relatively large amounts of surfactant; however, these attempts have achieved only limited success. Additionally, the usefulness of surfactants in large amounts can be limited by problems such as foaming, which can cause gas entrapment, and irritation of the gastrointestinal tract.

It is known to provide liquid dosage forms, including encapsulated liquid dosage forms, of poorly water-soluble drugs as self-emulsifying formulations. These formulations are generally designed to form an emulsion, in some cases a micro emulsion, when mixed with gastrointestinal fluid. Such self-emulsifying formulations can help to maintain the drug in solubilized form for a sufficient period of time to provide enhanced absorption but, even when formulated in this way, certain drugs still have a tendency to precipitate and/or crystallize in gastrointestinal fluid. Furthermore, high surfactant loadings are often necessary to provide acceptable self emulsifying behavior, with the attendant problems indicated above.

There is therefore a need in the art for improved liquid formulations of poorly water-soluble drugs, particularly for such formulations that are finely self-emulsifiable in gastrointestinal fluid. The term "finely self-emulsifiable" herein means capable of forming an emulsion wherein at least about 25% by volume of the emulsion particles have a diameter not greater than about 1 µm. Where emulsion particle size distribution includes a greater proportion of larger particles, it is believed that a greater tendency exists for drug particle aggregation and/or the potential for rapid absorption is reduced.

An illustrative class of drugs for which this need is apparent is the class of selective cyclooxygenase-2 (COX-2) inhibitory drugs of low water solubility.

Numerous compounds have been reported having therapeutically and/or prophylactically useful selective COX-2 inhibitory effect, and have been disclosed as having utility in treatment or prevention of specific COX-2 mediated disorders or of such disorders in general. Among such compounds are a large number of substituted pyrazolyl benzenesulfonamides as reported in U.S. Patent No. 5,466,823 to Talley et al*.,* including for example the compound 4-[5-(4-methylphenyl)-3-(trifluoromethyl)-1H-pyrazol-1-yl]benzenesulfonamide, also referred to herein as celecoxib (I), and the compound 4-[5-(3-fluoro-4-methoxyphenyl)-3-difluoromethyl)-1H-pyrazol-1-yl]benzenesulfonamide, also referred to herein as deracoxib (II).

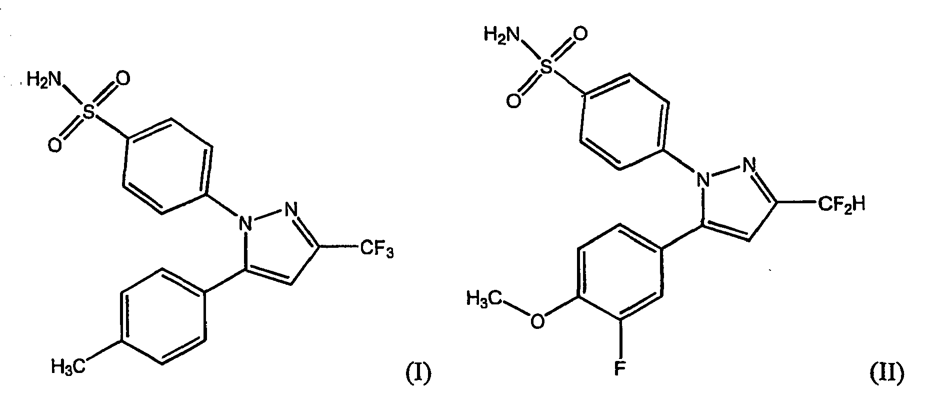

Other compounds reported to have therapeutically and/or prophylactically useful selective COX-2 inhibitory effect are substituted isoxazolyl benzenesulfonamides as reported in U.S. Patent No. 5,633,272 to Talley et al*.,* including the compound 4-[5-methyl-3-phenylisoxazol-4-yl]benzenesulfonamide, also referred to herein as valdecoxib (III).

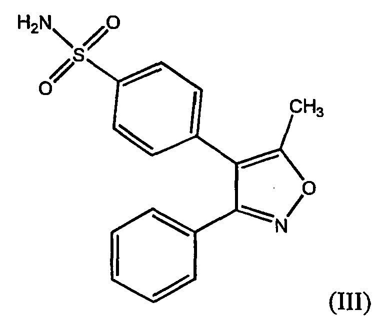

Still other compounds reported to have therapeutically and/or prophylactically useful selective COX-2 inhibitory effect are substituted (methylsulfonyl)phenyl furanones as reported in U.S. Patent No. 5,474,995 to Ducharme et al*.,* including the compound 3-phenyl-4-[4-(methylsulfonyl)phenyl]-5H-furan-2-one, also referred to herein as rofecoxib (IV).

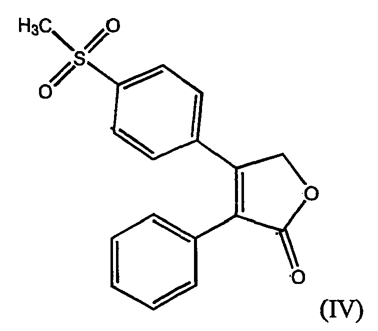

U.S. Patent No. 5,981,576 to Belley et al*.* discloses a further series of (methylsulfonyl)phenyl furanones said to be useful as selective COX-2 inhibitory drugs, including 3-(1-cyclopropylmethoxy)-5,5-dimethyl-4-[4-(methylsulfonyl)phenyl]-5H-furan-2-one and 3-(1-cyclopropylethoxy)-5,5-dimethyl-4-[4-(methylsulfonyl)phenyl]-5H-furan-2-one.

U.S. Patent No. 5,861,419 to Dube et al*.* discloses substituted pyridines said to be useful as selective COX-2 inhibitory drugs, including for example the compound 5-chloro-3-(4-methylsulfonyl)phenyl-2-(2-methyl-5-pyridinyl)pyridine, also referred to herein as etoricoxib (V).

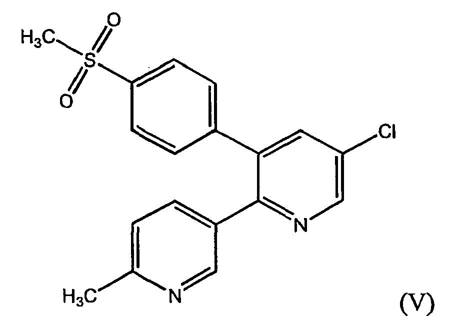

European Patent Application No. 0 863 134 discloses the compound 2-(3,5-difluorophenyl)-3-[4-(methylsulfonyl)phenyl]-2-cyclopenten-1-one said to be useful as a selective COX-2 inhibitory drug.

U.S. Patent No. 6,034,256 to Carter et al*.* discloses a series ofbenzopyrans said to be useful as selective COX-2 inhibitory drugs, including the compound (S)-6,8-dichloro-2-(trifluoromethyl)-2H-1-benzopyran-3-carboxylic acid (VI).

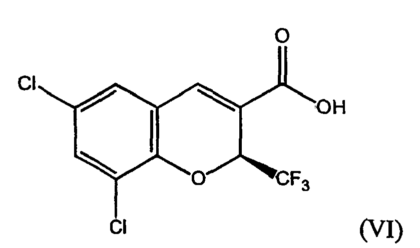

International Patent Publication No. WO 00/24719 discloses substituted pyridazinones said to be useful as selective COX-2 inhibitory drugs, including the compound 2-(3,4-difluorophenyl)-4-(3-hydroxy-3-methyl-1-butoxy)-5-[4-(methylsulfonyl)phenyl]-3-(2H)-pyridazinone.

A need for formulated compositions of selective COX-2 inhibitory drugs, particularly rapid-onset compositions of such drugs, exists. Rapid-onset drug delivery systems can provide many benefits over conventional dosage forms. Generally, rapid-onset preparations provide a more immediate therapeutic effect than standard dosage forms. For example, in the treatment of acute pain, for example in headache or migraine, rapid-onset dosage forms would be useful to provide fast pain relief.

Australian Patent Applications No. 200042711, No. 200043730 and No. 200043736 disclose compositions comprising a selective COX-2 inhibitory drug, a 5HT₁ receptor agonist and caffeine, said to be useful for treating migraine.

U.S. Patent No. 5,993,858 to Crison & Amidon discloses an excipient formulation for increasing bioavailability of a poorly water-soluble drug. The formulation is said to be self-microemulsifying and to comprise an oil or other lipid material, a surfactant and a hydrophilic co-surfactant. The choice of surfactant is said to be less critical than the choice of co-surfactant, which reportedly should have an HLB (hydrophilic-lipophilic balance) number greater than 8. A preferred example of such a co-surfactant is said to be Labrasol^{™} of Gattefossé, identified as a product "comprised of medium-chain triglycerides derived from coconut oil" having HLB of 14. A formulation prepared containing 15 mg nifedipine in a size 1 (0.5 ml) capsule, *i.e.*, at a concentration of 30 mg/ml, is described as a "clear solution" at 70°C but a "semi-solid" at room temperature.

Cited in above-referenced U.S. Patent No. 5,993,858 is prior work by Farah *et al.* in which a self-microemulsifying formulation was investigated for improving in vitro dissolution of indomethacin. The formulation of Farah *et al.* reportedly comprised an oil phase material Gelucire^{™} of Gattefossé Corporation, together with a polyethylene glycol capric/caprylic glyceride product having HLB of 10, a propylene glycol laurate product having HLB of 4, and diethylene glycol monoethyl ether.

Drugs of low water solubility are sometimes orally administered in suspension in an imbibable aqueous liquid. For example, a suspension ofparticulate celecoxib in a vehicle of apple juice is disclosed in co-assigned International Patent Publication No. WO 00/32189, incorporated herein by reference. Also disclosed therein is a dilute solution of celecoxib in a mixture of PEG-400 (polyethylene glycol having an average molecular weight of about 400) and water in a 2:1 ratio by volume.

The suspension and solution compositions of WO 00/32189 are indicated therein to have comparable bio availability. However, following oral administration to dogs, the time taken for blood serum celecoxib concentration to reach a maximum level (Tₘₐₓ) was shorter for the solution composition than for the suspension.

Above-cited U.S. Patent No. 5,760,068 discloses that its subject pyrazolyl benzenesulfonamide compounds, of which celecoxib and deracoxib are examples, can be administered parenterally as isotonic solutions in a range of solvents including polyethylene glycol and propylene glycol. It is also disclosed therein that the subject compounds can alternatively be present in a controlled-release capsule or tablet formulation for oral administration wherein, for example, such a compound is dispersed in hydroxypropylmethylcellulose (HPMC).

Above-cited U.S. Patent No. 5,633,272 discloses that its subject isoxazolyl benzenesulfonamides, of which valdecoxib is an example, can be administered parenterally as isotonic solutions in a range of solvents including polyethylene glycol and propylene glycol. It is also disclosed therein that the subject compounds can alternatively be present in a controlled-release capsule or tablet formulation for oral administration wherein, for example, such a compound is dispersed in HPMC.

Above-cited U.S. Patent No. 5,474,995 discloses that its subject (methylsulfonyl)phenyl furanones, of which rofecoxib is an example, can be administered parenterally in an isotonic solution in 1,3-butanediol. Also disclosed therein are oil-in-water emulsions, syrups and elixirs for oral administration, formulated with a sweetening agent such as propylene glycol, and aqueous suspensions formulated with suspending agents including methylcellulose and HPMC.

Above-cited U.S. Patent No. 5,861,419 discloses that its subject substituted pyridines, of which etoricoxib is an example, can be administered parenterally in an isotonic solution in 1,3-butanediol. Also disclosed therein are oil-in-water emulsions, syrups and elixirs for oral administration, formulated with a sweetening agent such as propylene glycol, and aqueous suspensions formulated with suspending agents including methylcellulose and HPMC.

International Patent Application WO 02/083177 describes finely self-emulsifiable pharmaceutical compositions comprising a low-stability drug, a fatty acid, and an organic amine. COX-2 inhibitory drugs are discussed.

International Patent Application WO 02/05799 describes self-emulsifiable pharmaceutical compositions comprising a COX-2 inhibitory drug, a fatty acid, and an organic amine.

Many selective COX-2 inhibitory compounds, including celecoxib, deracoxib, valdecoxib, rofecoxib and etoricoxib, have low solubility in aqueous media. In addition, some, for example celecoxib, have relatively high dose requirements. These properties present practical problems in formulating concentrated solutions of selective COX-2 inhibitory drugs for rapid-onset, oral administration. With respect to such high dose, low solubility drugs, the size of the capsule or volume of solution required to provide a therapeutic dose becomes a limiting factor. For example, a drug that has a solubility of 10 mg/ml in a given solvent and a therapeutic dose of 400 mg/day would require ingestion of 40 ml of solution. Such a volume can be inconvenient or unacceptable for consumption in imbibable form; this volume also presents particular problems where an encapsulated dosage form is desired because capsules that contain more than about 1.0 ml to about 1.5 ml of liquid are generally considered to be too large for comfortable swallowing. Thus, where a solution is administered in capsule form, multiple capsules would need to be ingested in order to provide the required dose. To avoid such problems, a solvent must be selected wherein the drug has relatively high solubility.

As described hereinbelow, treatment with selective COX-2 inhibitory drugs of low water solubility is indicated in a very wide array of COX-2 mediated disorders and conditions. Therefore, if an improved self-emulsifying formulation, particularly a finely self-emulsifying formulation, of such a drug could be provided, a significant advance would be realized in treatment of COX-2 mediated conditions and disorders, particularly in treatment of acute disorders where early relief from pain or other symptoms is desired. It would represent an especially important advance in the art to provide an effective method of treatment of acute pain, for example in headache or migraine, using such a formulation.

### SUMMARY OF THE INVENTION

There is now disclosed an orally deliverable pharmaceutical composition comprising a drug of low water solubility and a solvent liquid that comprises at least one pharmaceutically acceptable solvent, at least one pharmaceutically acceptable fatty acid and at least one pharmaceutically acceptable organic amine, wherein (a) a substantial portion, for example at least about 15% by weight, of the drug is in dissolved or solubilized form in the solvent liquid, and (b) the fatty acid and the organic amine are present in total and relative amounts such that the composition is finely self-emulsifiable in simulated gastric fluid.

A process for preparing such a composition is provided; the process comprises the steps of (a) providing at least one pharmaceutically acceptable fatty acid and at least one pharmaceutically acceptable organic amine; (b) providing a pharmaceutically acceptable solvent for the at least one fatty acid and a pharmaceutically acceptable solvent for the at least one organic amine; (c) admixing together with the at least one fatty acid, the solvent for the at least one fatty acid and the solvent for the at least one organic amine to form a pre-mix in which the fatty acid is substantially completely dissolved; (d) admixing together with the pre-mix the at least one organic amine to form a mixture in which the organic amine is substantially completely dissolved; and (e) admixing together with the mixture the drug of low water solubility in dissolved and/or solubilized form to form a pharmaceutical composition.

The term "solvent liquid" herein encompasses all of the components of the liquid medium in which a particular drug is dissolved or solubilized. Thus the "solvent liquid" includes not only one or more solvents, fatty acids and organic amines, but optionally additional excipients such as co-solvents, surfactants, co-surfactants, stabilizing agents, crystallization inhibitors, antioxidants, sweeteners, flavoring agents, colorants, etc.

In a presently preferred composition, substantially all of the drug is in dissolved or solubilized form in the solvent liquid and substantially none of the drug is in solid particulate form. Such a composition is referred to herein as a "solution".

An alternative composition comprises, in addition to a first portion of the drug in dissolved or solubilized form, a second portion of the drug in particulate form dispersed in the solvent liquid. In this embodiment, part of the drug is in solution and part is in suspension. Such a composition is referred to herein as a "solution/suspension".

"Simulated gastric fluid", abbreviated herein to "SGF", is an aqueous solution of 0.01M hydrochloric acid and 0.15M sodium chloride, having a pH of about 2.

In a presently preferred embodiment, the solution or solution/suspension is encapsulated in one or more capsules having a wall that breaks down in gastrointestinal fluid to release the drug within a short period of time after entry into the gastrointestinal tract.

Compositions are illustratively useful where the drug is a selective COX-2 inhibitory drug, and have been found to resolve at least some of the difficulties alluded to above in a surprisingly effective manner. Thus, according to the invention, a drug of low water solubility is now provided in a finely self-emulsifiable solution formulation. Preferably such a formulation is presented in a dosage form that is convenient for oral administration. Formulations of the invention are particularly advantageous because they permit a high concentration of the drug, are suitable for encapsulation and, following oral administration thereof, can permit rapid absorption of the drug into the bloodstream through formation of a fine emulsion in the aqueous environment of the gastrointestinal tract. By virtue of this rapid absorption, formulations of the invention can provide rapid onset of therapeutic action.

It can be theorized that a poorly water-soluble drug can provide more rapid onset of therapeutic effect when orally administered in solution, particularly a self-emulsifiable solution, than in particulate form because the process of dissolution in the gastrointestinal tract is not required. An even greater advantage by comparison with a solid formulation such as a tablet can be postulated because neither disintegration nor dissolution is required in the case of the solution composition.

Additionally, a drug administered in imbibable solution can be available for absorption higher in the alimentary tract, for example, in the mouth and esophagus, than one that becomes available for absorption only upon disintegration of the carrier formulation in the stomach or bowel.

A further advantage of liquid dosage forms such as imbibable solutions and solution/suspensions for many subjects is that these dosage forms are easy to swallow. A yet further advantage of imbibable liquid dosage forms is that metering of doses is continuously variable, providing infinite dose flexibility. The benefits of ease of swallowing and dose flexibility are particularly advantageous for infants, children and the elderly.

When encapsulated, a solution or solution/suspension can provide the subject with the beneficial rapid absorption characteristics associated with liquid formulations in addition to the convenience of a discrete, easy to swallow capsule form.

The highly concentrated solutions are beneficial for several reasons. First, concentrated solutions are less costly to package and easier to transport and handle than dilute solutions. Second, concentrated solutions provide flexibility in administration as they can be administered with any desired degree of dilution. And third, concentrated drug solutions, especially when encapsulated, do not require consumption of large volumes of fluid, which can be uncomfortable for many patient populations.

### DETAILED DESCRIPTION OF THE INVENTION

Novel processes according to the present invention are used for preparing finely self-emulsifiable pharmaceutical compositions which comprise one or more orally deliverable dose units. The term "orally deliverable" herein means suitable for oral administration. The term "oral administration" herein includes any form of delivery of a therapeutic agent or a composition thereof to a subject wherein the agent or composition is placed in the mouth of the-subject, whether or not the agent or composition is swallowed. Thus "oral administration" includes buccal and sublingual as well as esophageal administration. Absorption of the agent can occur in any part or parts of the gastrointestinal tract including the mouth, esophagus, stomach, duodenum, jejunum, ileum and colon. The term "dose unit" herein means a portion of a pharmaceutical composition that contains an amount of a therapeutic agent suitable for a single oral administration to provide a therapeutic effect. Typically one dose unit, or a small plurality (up to about 4) of dose units, provides a sufficient amount of the agent to result in the desired effect.

### Drug of low water solubility

Each dose unit or small plurality of dose units comprises, in a therapeutically and/or prophylactically effective total amount, a drug of low water solubility. A "drug of low water solubility" or "poorly water solubility drug" herein refers to any drug compound having a solubility in water, measured at 37°C, not greater than about 10 mg/ml, and preferably not greater than about 1 mg/ml. It is contemplated that compositions of the invention are especially advantageous for drugs having a solubility in water, measured at 37°C, not greater than about 0.1 mg/ml.

Solubility in water for many drugs can be readily determined from standard pharmaceutical reference books, for example The Merck Index, 11th ed., 1989 (published by Merck & Co., Inc., Rahway, NJ); the United States Pharmacopoeia, 24th ed. (USP 24), 2000; The Extra Pharmacopoeia, 29th ed., 1989 (published by Pharmaceutical Press, London); and the Physicians Desk Reference (PDR), 2001 ed. (published by Medical Economics Co., Montvale, NJ), each of which is individually incorporated herein by reference.

For example, individual drugs of low solubility as defined herein include those drugs categorized as "slightly soluble", "very slightly soluble", "practically insoluble" and "insoluble" in USP 24, pp. 2254-2298; and those drugs categorized as requiring 100 ml or more of water to dissolve 1 g of the drug, as listed in USP 24, pp. 2299-2304.

Illustratively, suitable drugs of low water solubility include, without limitation, drugs from the following classes: abortifacients, ACE inhibitors, α- and β-adrenergic agonists, α- and β-adrenergic blockers, adrenocortical suppressants, adrenocorticotropic hormones, alcohol deterrents, aldose reductase inhibitors, aldosterone antagonists, anabolics, analgesics (including narcotic and non-narcotic analgesics), androgens, angiotensin II receptor antagonists, anorexics, antacids, anthelminthics, antiacne agents, antiallergics, antialopecia agents, antiamebics, antiandrogens, antianginal agents, antiarrhythmics, antiarteriosclerotics, antiarthritic/antirheumatic agents (including selective COX-2 inhibitors), antiasthmatics, antibacterials, antibacterial adjuncts, anticholinergics, anticoagulants, anticonvulsants, antidepressants, antidiabetics, antidiarrheal agents, antidiuretics, antidotes to poison, antidyskinetics, antieczematics, antiemetics, antiestrogens, antifibrotics, antiflatulents, antifungals, antiglaucoma agents, antigonadotropins, antigout agents, antihistaminics, antihyperactives, antihyperlipoproteinemics, antihyperphosphatemics, antihypertensives, antihyperthyroid agents, antihypotensives, antihypothyroid agents, anti-inflammatories, antimalarials, antimanics, antimethemoglobinemics, antimigraine agents, antimuscarinics, antimycobacterials, antineoplastic agents and adjuncts, antineutropenics, antiosteoporotics, antipagetics, antiparkinsonian agents, antipheochromocytoma agents, antipneumocystis agents, antiprostatic hypertrophy agents, antiprotozoals, antipruritics, antipsoriatics, antipsychotics, antipyretics, antirickettsials; antiseborrheics, antiseptics/disinfectants, antispasmodics, antisyphylitics, antithrombocythemics, antithrombotics, antitussives, antiulceratives, antiurolithics, antivenins, antiviral agents, anxiolytics, aromatase inhibitors, astringents, benzodiazepine antagonists, bone resorption inhibitors, bradycardic agents, bradykinin antagonists, bronchodilators, calcium channel blockers, calcium regulators, carbonic anhydrase inhibitors, cardiotonics, CCK antagonists, chelating agents, cholelitholytic agents, choleretics, cholinergics, cholinesterase inhibitors, cholinesterase reactivators, CNS stimulants, contraceptives, debriding agents, decongestants, depigmentors, dermatitis herpetiformis suppressants, digestive aids, diuretics, dopamine receptor agonists, dopamine receptor antagonists, ectoparasiticides, emetics, enkephalinase inhibitors, enzymes, enzyme cofactors, estrogens, expectorants, fibrinogen receptor antagonists, fluoride supplements, gastric and pancreatic secretion stimulants, gastric cytoprotectants, gastric proton pump inhibitors, gastric secretion inhibitors, gastroprokinetics, glucocorticoids, α-glucosidase inhibitors, gonad-stimulating principles, growth hormone inhibitors, growth hormone releasing factors, growth stimulants, hematinics, hematopoietics, hemolytics, hemostatics, heparin antagonists, hepatic enzyme inducers, hepatoprotectants, histamine H₂ receptor antagonists, HIV protease inhibitors, HMG CoA reductase inhibitors, immunomodulators, immunosuppressants, insulin sensitizers, ion exchange resins, keratolytics, lactation stimulating hormones, laxatives/cathartics, leukotriene antagonists, LH-RH agonists, lipotropics, 5-lipoxygenase inhibitors, lupus erythematosus suppressants, matrix metalloproteinase inhibitors, mineralocorticoids, miotics, monoamine oxidase inhibitors, mucolytics, muscle relaxants, mydriatics, narcotic antagonists, neuroprotectives, nootropics, ovarian hormones, oxytocics, pepsin inhibitors, pigmentation agents, plasma volume expanders, potassium channel activators/openers, progestogens, prolactin inhibitors, prostaglandins, protease inhibitors, radio-pharmaceuticals, 5α-reductase inhibitors, respiratory stimulants, reverse transcriptase inhibitors, sedatives/hypnotics, serenics, serotonin noradrenaline reuptake inhibitors, serotonin receptor agonists, serotonin receptor antagonists, serotonin uptake inhibitors, somatostatin analogs, thrombolytics, thromboxane A₂ receptor antagonists, thyroid hormones, thyrotropic hormones, tocolytics, topoisomerase I and II inhibitors, uricosurics, vasomodulators including vasodilators and vasoconstrictors, vasoprotectants, xanthine oxidase inhibitors, and combinations thereof

Non-limiting illustrative examples of suitable drugs of low water solubility include, for example, acetohexamide, acetylsalicylic acid, alclofenac, allopurinol, atropine, benzthiazide, carprofen, celecoxib, chlordiazepoxide, chlorpromazine, clonidine, codeine, codeine phosphate, codeine sulfate, deracoxib, diacerein, diclofenac, diltiazem, estradiol, etodolac, etoposide, etoricoxib, fenbufen, fenclofenac, fenprofen, fentiazac, flurbiprofen, griseofulvin, haloperidol, ibuprofen, indomethacin, indoprofen, ketoprofen, lorazepam, medroxyprogesterone acetate, megestrol, methoxsalen, methylprednisone, morphine, morphine sulfate, naproxen, nicergoline, nifedipine, niflumic, oxaprozin, oxazepam, oxyphenbutazone, paclitaxel, phenindione, phenobarbital, piroxicam, pirprofen, prednisolone, prednisone, procaine, progesterone, pyrimethamine, rofecoxib, sulfadiazine, sulfamerazine, sulfisoxazole, sulindac, suprofen, ternazepam, tiaprofenic acid, tilomisole, tolmetic, valdecoxib, etc.

The amount of drug incorporated in a dosage form can be selected according to known principles of pharmacy. A therapeutically effective amount of drug is specifically contemplated. The term "therapeutically and/or prophylactically effective amount" as used herein refers to an amount of drug that is sufficient to elicit the required or desired therapeutic and/or prophylactic response.

In a particularly preferred embodiment, the drug is a selective COX-2 inhibitory drug of low water solubility. Any such selective COX-2 inhibitory drug known in the art can be used. Compositions are especially useful for compounds of formula (VII) 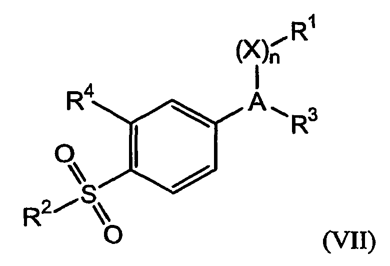 wherein:
- A: is a substituent selected from partially unsaturated or unsaturated heterocyclyl and partially unsaturated or unsaturated carbocyclic rings, preferably a heterocyclyl group selected from pyrazolyl, furanonyl, isoxazolyl, pyridinyl, cyclopentenonyl and pyridazinonyl groups;
- X: is O, S or CH₂;
- n: is 0 or 1;
- R¹ is: at least one substituent selected from heterocyclyl, cycloalkyl, cycloalkenyl and aryl, and is optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio;
- R²: is methyl, amino or aminocarbonylalkyl;
- R³: is one or more radicals selected from hydrido, halo, alkyl, alkenyl, alkynyl, oxo, cyano, carboxyl, cyanoalkyl, heterocyclyloxy, alkyloxy, alkylthio, alkylcarbonyl, cycloalkyl, aryl, haloalkyl, heterocyclyl, cycloalkenyl, aralkyl, heterocyclylalkyl, acyl, alkylthioalkyl, hydroxyalkyl, alkoxycarbonyl, arylcarbonyl, aralkylcarbonyl, aralkenyl, alkoxyalkyl arylthioalkyl, aryloxyalkyl, aralkylthioalkyl, aralkoxyalkyl, alkoxyaralkoxyalkyl, alkoxycarbonylalkyl, aminocarbonyl, aminocarbonylalkyl, alkylaminocarbonyl, N-arylaminocarbonyl, N-alkyl-N-arylaminocarbonyl, alkylaminocarbonylalkyl, carboxyalkyl, alkylamino, N-arylamino, N-aralkylamino, N-alkyl-N-aralkylamino, N-alkyl-N-arylamino, aminoalkyl, alkylaminoalkyl, N-arylaminoalkyl, N-aralkylaminoalkyl, N-alkyl-N-aralkylaminoalkyl, N-alkyl-N-arylaminoalkyl, aryloxy, aralkoxy, arylthio, aralkylthio, alkylsulfinyl, alkylsulfonyl, aminosulfonyl, alkylaminosulfonyl, N-arylaminosulfonyl, arylsulfonyl and N-alkyl-N-arylaminosulfonyl, R³ being optionally substituted at a substitutable position with one or more radicals selected from alkyl, haloalkyl, cyano, carboxyl, alkoxycarbonyl, hydroxyl, hydroxyalkyl, haloalkoxy, amino, alkylamino, arylamino, nitro, alkoxyalkyl, alkylsulfinyl, halo, alkoxy and alkylthio; and
- R⁴: is selected from hydrido and halo.

Compositions are especially useful for selective COX-2 inhibitory drugs having the formula (VIII): 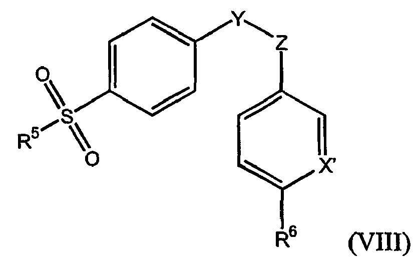 where R⁵ is a methyl or amino group, R⁶ is hydrogen or a C₁₋₄ alkyl or alkoxy group, X' is N or CR⁷ where R⁷ is hydrogen or halogen, and Y and Z are independently carbon or nitrogen atoms defining adjacent atoms of a five- to six-membered ring that is optionally substituted at one or more positions with oxo, halo, methyl or halomethyl groups, or an isomer, tautomer, pharmaceutically-acceptable salt or prodrug thereof. Preferred such five- to six-membered rings are cyclopentenone, furanone, methylpyrazole, isoxazole and pyridine rings substituted at no more than one position.

Illustratively, compositions are suitable for celecoxib, deracoxib, valdecoxib, rofecoxib, etoricoxib, 2-(3,5-difluorophenyl)-3-[4-(methylsulfonyl)phenyl]-2-cyclopenten-1-one and 2-(3,4-difluorophenyl)-4-(3-hydroxy-3-methyl-1-butoxy)-5-[4-(methylsulfonyl)phenyl]-3-(2H)-pyridazinone.

Compositions are also useful for compounds having the formula (IX): 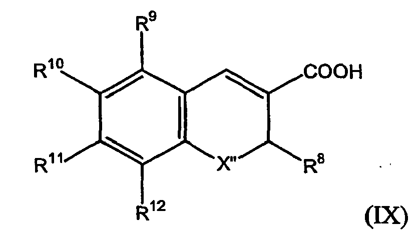 where X" is O, S or N-lower alkyl; R⁸ is lower haloalkyl; R⁹ is hydrogen or halogen; R¹⁰ is hydrogen, halogen, lower alkyl, lower alkoxy or haloalkoxy, lower aralkylcarbonyl, lower dialkylaminosulfonyl, lower alkylaminosulfonyl, lower aralkylaminosulfonyl, lower heteroaralkylaminosulfonyl, or 5- or 6- membered nitrogen-containing heterocyclosulfonyl; and R¹¹ and R¹² are independently hydrogen, halogen, lower alkyl, lower alkoxy, or aryl; and for pharmaceutically acceptable salts thereof.

A particularly useful compound of formula (IX) is (S)-6,8-dichloro-2-(trifluoromethyl)-2H-1-benzopyran-3-carboxylic acid.

Illustratively, celecoxib, deracoxib, valdecoxib, rofecoxib, etoricoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid and 2-(3,4-difluorophenyl)-4-(3-hydroxy-3-methyl-1-butoxy)-5-[4-(methylsulfonyl)phenyl]-3-(2H)-pyridazinone, more particularly celecoxib, valdecoxib, rofecoxib and etoricoxib, and still more particularly celecoxib and valdecoxib, are useful in the method and composition of the invention.

The invention is illustrated herein with particular reference to celecoxib, and it will be understood that any other selective COX-2 inhibitory drug of low solubility in water can, if desired, be substituted in whole or in part for celecoxib in compositions herein described. For example, compositions are suitable for formulation of valdecoxib, alone or in combination with celecoxib.

Where the drug is celecoxib, the composition typically comprises celecoxib in a therapeutically and/or prophylactically effective total amount of about 10 mg to about 1000 mg per dose unit. Where the drug is a selective COX-2 inhibitory drug other than celecoxib, the amount of the drug per dose unit is therapeutically equivalent to about 10 mg to about 1000 mg of celecoxib.

It will be understood that a therapeutically and/or prophylactically effective amount of a drug for a subject is dependent *inter alia* on the body weight of the subject. A "subject" herein to which a therapeutic agent or composition thereof can be administered includes a human patient of either sex and of any age, and also includes any nonhuman animal, particularly a domestic or companion animal, illustratively a cat, dog or horse.

Where the subject is a child or a small animal (*e.g.*, a dog), for example, an amount of celecoxib relatively low in the preferred range of about 10 mg to about 1000 mg is likely to be consistent with therapeutic effectiveness. Where the subject is an adult human or a large animal (*e.g.*, a horse), therapeutic effectiveness is likely to require dose units containing a relatively greater amount of celecoxib. For an adult human, a therapeutically effective amount of celecoxib per dose unit in a composition of the present invention is typically about 10 mg to about 400 mg. Especially preferred amounts of celecoxib per dose unit are about 100 mg to about 200 mg, for example about 100 mg or about 200 mg.

For other selective COX-2 inhibitory drugs, an amount of the drug per dose unit can be in a range known to be therapeutically effective for such drugs. Preferably, the amount per dose unit is in a range providing therapeutic equivalence to celecoxib in the dose ranges indicated immediately above.

### Form of compositions

Compositions are preferably in the form of a concentrated solution that may or may not be encapsulated as a discrete article. If encapsulated, preferably a single such article or a small plurality (up to about 10, more preferably no more than about 4) of such articles is sufficient to provide the daily dose. Alternatively, compositions are in the form of a concentrated imbibable liquid. The phrase "imbibable liquid" is used herein to refer to an unencapsulated substantially homogeneous flowable mass, such as a solution or solution/suspension, administered orally and swallowed in liquid form and from which single dose units are measurably removable. The term "substantially homogeneous" with reference to a pharmaceutical composition that comprises several components means that the components are sufficiently mixed such that individual components are not present as discrete layers and do not form concentration gradients within the composition.

A particular dose unit can be selected to accommodate the desired frequency of administration used to achieve a specified daily dose. For example, a daily dosage amount of 400 mg can be accommodated by administration of one 200 mg dose unit, or two 100 mg dose units, twice a day. The amount of the composition that is administered and the dosage regimen for treating the condition or disorder will depend on a variety of factors, including the age, weight, sex and medical condition of the subject, the nature and severity of the condition or disorder, the route and frequency of administration, and the particular drug selected, and thus may vary widely. It is contemplated, however, that for most purposes a once-a-day or twice-a-day administration regimen provides the desired therapeutic efficacy.

A composition comprises a drug of low water solubility, at least a portion of which is in dissolved or solubilized form in a solvent liquid suitable for oral administration.

The solvent liquid comprises at least one pharmaceutically acceptable solvent, at least one pharmaceutically acceptable fatty acid and at least one pharmaceutically acceptable organic amine, and optionally one or more additional components, including pharmaceutically acceptable excipients. The term "excipient" herein means any substance, not itself a therapeutic agent, used as a carrier or vehicle for delivery of a therapeutic agent to a subject or added to a pharmaceutical composition to improve its handling, storage, disintegration, dispersion, dissolution, release or organoleptic properties or to permit or facilitate formation of a dose unit of the composition into a discrete article such as a capsule suitable for oral administration. Excipients can include, by way of illustration and not limitation, diluents, disintegrants, dispersants, binding agents, adhesives, wetting agents, lubricants, glidants, crystallization inhibitors, stabilizers, antioxidants, substances added to mask or counteract a disagreeable taste or odor, flavors, dyes, fragrances, preservatives, and substances added to improve appearance of the composition.

Such optional additional components should be physically and chemically compatible with the other ingredients of the composition and should not be deleterious to the recipient. Importantly, some of the above-listed classes of excipients overlap each other. Compositions of the present invention can be adapted for administration by any suitable oral route by selection of appropriate solvent liquid components and a dosage of the drug effective for the treatment intended. Accordingly, components employed in the solvent liquid can themselves be solids, semi-solids, liquids, or combinations thereof.

An imbibable composition can be in the form of, for example, a solution, a solution/suspension, an elixir, a syrup, or any other liquid form reasonably adapted for oral administration. Such compositions can also comprise excipients selected from, for example, emulsifying and suspending agents, sweetening and flavoring agents, surfactants and co-surfactants.

Alternatively, as described in detail below, a composition can be prepared in the form of discrete unit dose articles, for example, capsules having a wall that illustratively comprises gelatin and/or a cellulosic polymer such as HPMC, each capsule containing a liquid composition comprising a predetermined amount of drug in a solvent liquid. The liquid composition within the capsule is released by breakdown of the wall on contact with gastrointestinal fluid. The particular mechanism of capsule wall breakdown is not important and can include such mechanisms as erosion, degradation, dissolution, etc.

The compositions comprise a therapeutically effective amount of a drug of low water solubility, for example celecoxib or valdecoxib, substantially completely dissolved in a pharmaceutically acceptable solvent liquid comprising at least one solvent, at least one fatty acid and at least one organic amine. Substantially no part of the drug is present in solid particulate form. Compositions can be formulated either in an imbibable or discrete dosage form (*e.g.*, encapsulated). Such compositions optionally further comprise a crystallization inhibitor as more fully described below, the crystallization inhibitor being present in the solvent liquid and/or as a component of a capsule wall. Preferably, concentrated solutions of this embodiment have a drug concentration of about 10% to about 75%, more preferably about 20% to about 75%, by weight of the composition.

### Solvent

A preferred solvent is a glycol or glycol ether. Suitable glycol ethers include those conforming to formula (X):

R¹—O—((CH₂)ₘO)ₙ— R² (X)

wherein R¹ and R² are independently hydrogen or C₁₋₆ akyl, C₁₋₆ akenyl, phenyl or benzyl groups, but no more than one of R¹ and R² is hydrogen; m is an integer of 2 to about 5; and n is an integer of 1 to about 20. It is preferred that one of R¹ and R² is a C₁₋₄ alkyl group and the other is hydrogen or a C₁₋₄ alkyl group; more preferably at least one of R¹ and R² is a methyl or ethyl group. It is preferred that m is 2. It is preferred that n is an integer of 1 to about 4, more preferably 2.

Glycol ethers used as solvents in compositions of the present invention typically have a molecular weight of about 75 to about 1000, preferably about 75 to about 500, and more preferably about 100 to about 300. Importantly, the glycol ethers used in compositions of the present invention must be pharmaceutically acceptable and must meet all other conditions prescribed herein.

Non-limiting examples of glycol ethers that may be used in compositions of the present invention include ethylene glycol monomethyl ether, ethylene glycol dimethyl ether, ethylene glycol monoethyl ether, ethylene glycol diethyl ether, ethylene glycol monobutyl ether, ethylene glycol dibutyl ether, ethylene glycol monophenyl ether, ethylene glycol monobenzyl ether, ethylene glycol butylphenyl ether, ethylene glycol terpinyl ether, diethylene glycol monomethyl ether, diethylene glycol dimethyl ether, diethylene glycol monoethyl ether, diethylene glycol diethyl ether, diethylene glycol divinyl ether, ethylene glycol monobutyl ether, diethylene glycol dibutyl ether, diethylene glycol monoisobutyl ether, triethylene glycol dimethyl ether, triethylene glycol monoethyl ether, triethylene glycol monobutyl ether, tetraethylene glycol dimethyl ether, and mixtures thereof. See for example Flick (1998): Industrial Solvents Handbook, 5th ed., Noyes Data Corporation, Westwood, NJ. A particularly suitable glycol ether solvent is diethylene glycol monoethyl ether, sometimes referred to in the art as DGME or ethoxydiglycol. It is available for example under the trademark Transcutol^{™} of Gattefossé Corporation.

Glycols suitable as solvents in compositions of the present invention include propylene glycol, 1,3-butanediol and polyethylene glycols. A presently preferred solvent is polyethylene glycol (PEG).

Any pharmaceutically acceptable PEG can be used. Preferably, the PEG has an average molecular weight of about 100 to about 10,000, and more preferably about 100 to about 1,000. Still more preferably, the PEG is of liquid grade. Non-limiting examples of PEGs that can be used in solvent liquids of this invention include PEG-200, PEG-350, PEG-400, PEG-540 and PEG-600. See for example Flick (1998), *op. cit.,* p. 392. A presently preferred PEG has an average molecular weight of about 375 to about 450, as exemplified by PEG-400.

PEGs such as PEG-400 have many desirable properties as solvents for poorly water-soluble drugs. In the case of celecoxib, for example, the drug can be dissolved or solubilized at a very high concentration in PEG-400, enabling formulation of a therapeutically effective dose in a very small volume of solvent liquid. This is especially important where the resulting solution is to be encapsulated, as capsules of a size convenient for swallowing can be prepared containing a therapeutically effective dose even of a drug such as celecoxib having a relatively high dose requirement for efficacy. Importantly, ethanol, water, and other excipients identified as co-solvents hereinbelow or elsewhere can, if desired, be used as solvents in a composition of the invention. Typically, one or more solvents will be present in a composition of the invention in a total amount of about 5% to about 95%, preferably about 10% to about 90% and more preferably about 15% to about 85%, by weight. However, a solvent alone, even a very good solvent such as PEG, is not sufficient to provide a finely self emulsifiable formulation. According to the present invention and as described more fully below, a combination of a fatty acid and an amine, preferably an organic amine, provide a surprisingly effective solution to the problem of providing a finely self emulsifiable liquid formulation of a poorly water-soluble drug. Therefore, in a particularly preferred embodiment, the solvent liquid comprises a pharmaceutically acceptable solvent for the at least one fatty acid and a pharmaceutically acceptable solvent for the at least one organic amine. The term "a pharmaceutically acceptable solvent for the at least one fatty acid" means that the solvent should be capable of dissolving relevant quantities of the fatty acid, preferably with moderate stirring at room temperature. The term "a pharmaceutically acceptable solvent for the at least one organic amine" means that the solvent should be capable of dissolving relevant quantities of the organic amine, preferably with moderate stirring at room temperature. A technician will, through routine experimentation, readily identify pharmaceutically acceptable solvent(s) for the fatty acid and for the organic amine. In some cases, a solvent may be an acceptable solvent for both the organic amine and the fatty acid while in other cases, more than one solvent will be used.

### Fatty acid and organic amine

Inclusion of a combination of a fatty acid and an organic amine in a solution or solution/suspension composition of a poorly water-soluble drug can render the composition finely self-emulsifiable in SGF. Therefore, a composition of the invention comprises at least one pharmaceutically acceptable fatty acid and at least one pharmaceutically acceptable amine, preferably an organic amine (also referred to herein as a "fatty acid/organic amine pair"). Without being bound by theory, it is believed that a fatty acid/organic amine pair, when present in appropriate total and relative amounts in the solvent liquid, promotes formation of charged fine-emulsion droplets upon exposure of the composition to an aqueous medium such as SGF.

Whether a composition is "finely self-emulsifiable" in SGF as defined herein can illustratively be determined according to Test I.

### Test I:

A. A 400 µl aliquot of a test composition is placed into a screw-top, side-arm vessel containing 20 ml SGF (maintained at 37°C throughout the test) to form a test liquid.
B. The test liquid is mildly agitated at 75 rpm for 2 minutes using an orbital shaker, to permit emulsification.
C. A 5—50 µl aliquot of the test liquid is withdrawn through the side-arm using a pipette and is discharged from the pipette into a sampling vessel.
D. A pump (e.g., model RH0CKC-LF, Fluid Metering Inc., Syosset, NY) is used to pull the test liquid from the sampling vessel through a combination scattering/obscuration sensor (e.g., LE400-0.5, Particle Sizing Systems, Santa Barbara, CA) at a rate of 1 ml/minute for a period of 1 minute.
E. Emulsion particles are counted individually by light scattering in the size (i.e., diameter) range from 0.5 to 1 µm and by light obscuration in the size range above 1 µm, using the vendor's software (*e.g.*, Version 1.59).
F. A plot is prepared of number (*i.e.,* unweighted) or volume (*i.e.,* weighted) of emulsion particles versus particle diameter.
G. Integration of the plot, accounting for all dilutions, is performed to estimate total number or volume of emulsion particles present in the test liquid large enough to be detected by the sensor.
H. If Test I results in about 25% or more, by volume, of emulsion particles having a diameter of 1 µm or less, the test composition is deemed to be finely self-emulsifiable.

Preferred fatty acids have a saturated or unsaturated C₆₋₂₄ carbon chain. Non-limiting examples of suitable fatty acids include oleic acid, octanoic acid, caproic acid, caprylic acid, capric acid, eleostearic acid, lauric acid, myristic acid, palmitic acid, stearic acid, icosanoic acid, elaidic acid, linoleic acid, linolenic acid, eicosapentaenoic acid and docosahexaenoic acid. Oleic acid is an especially preferred fatty acid.

Preferred organic amines have a C₂₋₈ carbon chain with one or two amine groups. More preferably, organic amines can be selected from C₂₋₈ alkyl amines, alkylene diamines, alkanol amines, alkylalkanol amines, glycol ether amines and aryl amines. Non-limiting examples of suitable organic amines include monoethanolamine, diethanolamine, triethanolamine, dimethylaminoethanol, tromethamine, *etc*. Particularly preferred organic amines are tertiary amines, for example triethanolamine and dimethylaminoethanol.

A fatty acid/organic amine pair is selected (as to both type and amount of each component) such that when a composition of the invention is subjected to Test I, at least about 50%, more preferably at least about 75%, by volume of the emulsion particles counted have a diameter of about 1 µm or less. It is especially preferred that a substantial portion by volume of the emulsion particles counted, more preferably at least about 75%, still more preferably at least about 85%, and most preferably at least about 90%, have a diameter of about 0.5 µm or less.

A preferred mole ratio of fatty acid to amine group(s) in the organic amine is about 5:1 to about 1:100, more preferably about 3:1 to about 1:50, and still more preferably about 2:1 to about 1:10, for example about 1:1. Preferably, if present, the fatty acid and organic amine are collectively present in an amount of about 1% to about 50%, more preferably about 2% to about 30%, and still more preferably about 5% to about 15%, by weight of the composition.

It is believed, without being bound by theory, that a finely self-emulsifiable solution composition of the invention, particularly one having a fatty acid/organic amine pair as described above, will provide the drug in a form that is especially rapidly absorbable in the gastrointestinal tract.

### Other excipients

Compositions optionally contain pharmaceutically acceptable excipients other than a solvent and a crystallization inhibitor. In the case of a solution composition, for example, such excipients can include co-solvents, sweeteners, antioxidants, preservatives, dispersants, emulsifying agents, etc. Through selection and combination of excipients, compositions can be provided exhibiting improved performance with respect to drug concentration, dissolution, dispersion, emulsification, efficacy, flavor, patient compliance and other properties.

A composition, particularly a solution composition, optionally comprises one or more pharmaceutically acceptable co-solvents. Non-limiting examples of suitable co-solvents include additional glycols, alcohols, for example ethanol and n-butanol; oleic and linoleic acid triglycerides, for example soybean oil; caprylic/capric triglycerides, for example Miglyol^{™} 812 of Huls; caprylic/capric mono- and diglycerides, for example Capmul^{™} MCM of Abitec; polyoxyethylene caprylic/capric glycerides such as polyoxyethylene (8) caprylic/capric mono- and diglycerides, for example Labrasol^{™} of Gattefossé; propylene glycol fatty acid esters, for example propylene glycol laurate; polyoxyethylene (35) castor oil, for example Cremophor^{™} EL of BASF; polyoxyethylene glyceryl trioleate, for example Tagat^{™} TO of Goldschmidt; lower alkyl esters of fatty acids, for example ethyl butyrate, ethyl caprylate and ethyl oleate; and water.

In a solution composition, the drug, even when finely emulsified, can, upon exposure to the aqueous environment of the gastrointestinal tract, precipitate and agglomerate in a solid, typically crystalline, particulate form. Such precipitation and/or crystallization can adversely impact any rapid-onset benefits obtained by administering a drug in dissolved form, because a drug that has reverted to a crystalline form must undergo the process of dissolution prior to absorption.

Therefore, preferred compositions further comprise a crystallization inhibitor, also referred to herein as a turbidity-decreasing polymer. We have discovered that certain polymers can substantially inhibit precipitation and/or crystallization of a poorly water-soluble drug, when a solution of the drug in a substantially non-aqueous solvent is exposed to SGF. Accordingly, compositions of the present invention preferably comprise a turbidity-decreasing polymer. The polymer can be a cellulosic or non-cellulosic polymer and is preferably substantially water-soluble.

It will be understood that certain polymers are more effective at inhibiting precipitation and/or crystallization of a selected poorly water soluble drug than others, and that not all polymers inhibit precipitation and/or crystallization as described herein of every poorly water-soluble drug. Whether a particular polymer is useful as a crystallization inhibitor for a particular poorly water soluble drug according to the present invention can be readily determined by one of ordinary skill in the art, for example according to Test II.

### Test II:

A. A suitable amount of the drug is dissolved in a solvent (e.g., ethanol, dimethyl sulfoxide or, where the drug is an acid or base, water) to obtain a concentrated drug solution.
B. A volume of water or buffered solution with a fixed pH is placed in a first vessel and maintained at room temperature.
C. An aliquot of the concentrated drug solution is added to the contents of the first vessel to obtain a first sample solution having a desired target drug concentration. The drug concentration selected should be one which produces substantial precipitation and consequently higher apparent absorbance (i.e., turbidity) than a saturated solution having no such precipitation.
D. A test polymer is selected and, in a second vessel, the polymer is dissolved in water or a buffered solution with a fixed pH (identical in composition, pH and volume to that used in step C) in an amount sufficient to form a 0.25-2% w/w polymer solution.
E. To form a second sample solution, an aliquot of the concentrated drug solution prepared in step A is added to the polymer solution in the second vessel to form a sample solution having a final drug concentration equal to that of the first sample solution.
F. At 60 minutes after preparation of both sample solutions, apparent absorbance (*i.e.,* turbidity) of each sample solution is measured using light having a wavelength of 650 nm;
G. If the turbidity of the second sample solution is less than the turbidity of the first sample solution, the test polymer is deemed to be a "turbidity-decreasing polymer" and is useful as a crystallization inhibitor for the test drug.

A technician performing Test II will readily find a suitable polymer concentration for the test within the polymer concentration range provided above, by routine experimentation. In a particularly preferred embodiment, a concentration of the polymer is selected such that when Test II is performed, the apparent absorbance of the second sample solution is not greater than about 50% of the apparent absorbance of the first sample solution.

In another embodiment, compositions comprise a crystallization inhibitor comprising at least one cellulosic polymer. Preferred cellulosic polymers are selected from HPMC, methylcellulose, ethylcellulose, sodium carboxymethylcellulose and hydroxypropylcellulose. More preferably, the at least one cellulosic polymer is selected from cellulosic polymers having at least a portion of substitutable hydroxyl groups substituted with methoxyl and/or hydroxypropoxyl groups. Still more preferably, the at least one cellulosic polymer is HPMC.

HPMC useful as a crystallization inhibitor according to the invention preferably has a viscosity, 2% in water, of about 100 to about 20,000 cP. HPMCs vary in the degree of substitution of available hydroxyl groups on the cellulosic backbone by methoxyl groups and by hydroxypropoxyl groups. With increasing hydroxypropoxyl substitution, the resulting HPMC becomes more hydrophilic in nature. It is preferred to use HPMC having about 15% to about 35%, more preferably about 19% to about 30%, and most preferably about 19% to about 24%, methoxyl substitution, and having about 3% to about 15%, more preferably about 4% to about 12%, and most preferably about 7% to about 12%, hydroxypropoxyl substitution.

Suitable HPMCs that are relatively hydrophilic in nature are illustratively available under the brand names Methocel^{™} of Dow Chemical Co. and Metolose^{™} of Shin-Etsu Chemical Co.

An illustrative presently preferred HPMC is one with substitution type 2208, denoting about 19% to about 24% methoxyl substitution and about 7% to about 12% hydroxypropoxyl substitution, and with a nominal viscosity, 2% in water, of about 4000 cP.

Surprisingly, it has been found that the crystallization inhibitor need not be a component of the solvent liquid. Optionally, a crystallization inhibitor such as HPMC can be a component of a capsule wall wherein a solution composition of the invention is encapsulated. In one embodiment, substantially no HPMC or other crystallization inhibitor is present in the solvent liquid but the capsule wall comprises HPMC. The capsule wall can even consist predominantly of HPMC.

If present, the crystallization inhibitor is preferably present in a total amount sufficient to substantially inhibit drug crystallization and/or precipitation upon dilution of the composition in SGF. An amount sufficient to "substantially inhibit drug crystallization and/or precipitation" herein means an amount sufficient to prevent, slow, inhibit or delay precipitation of drug from solution and/or to prevent, slow, inhibit or delay formation of crystalline drug particles from dissolved drug particles. For practical purposes, whether an amount of crystallization inhibitor in a given test composition is sufficient to substantially inhibit drug crystallization and/or precipitation can be determined according to Test III, which can also be used to determine whether a particular polymer component is useful as a crystallization inhibitor in a particular composition of the invention.

### Test III:

A. A volume of a test composition, either in unencapsulated or encapsulated form, having a polymer component is placed in a volume of SGF to form a mixture having a fixed ratio of about 1 g to about 2 g of the composition per 100 ml of SGF.
B. The mixture is maintained at a constant temperature of about 37°C and is stirred using type II paddles (USP 24) at a rate of 75 rpm for a period of 4 hours.
C. At one or more time-points after at least about 15 minutes of stirring but before about 4 hours of stirring, an aliquot of the mixture is drawn and filtered, for example through a non-sterile Acrodisc^{™} syringe filter with a 0.8 µm Versapor^{™} membrane.
D. Filtrate is collected in a vessel.
E. Drug concentration in the filtrate is measured using high performance liquid chromatography (HPLC).
F. The test is repeated identically with a comparative composition that is substantially similar to the test composition except that it lacks the polymer component. Where the polymer component in the test composition is present in the solvent liquid, it is replaced in the comparative composition by polyethylene glycol solvent. Where the polymer component in the test composition is present in a capsule wall, it is replaced in the comparative composition with gelatin.
G. If the drug concentration in the filtrate resulting from the test composition is greater than that in the filtrate resulting from the comparative composition, the polymer component present in the test composition is deemed to substantially inhibit crystallization and/or precipitation of the drug in SGF.

A crystallization inhibitor such as HPMC, when present in the solvent liquid, is generally present in a total amount of about 1% to about 20%, preferably about 1% to about 15%, and most preferably about 1% to about 10%, by weight of the solvent liquid. Typically, the higher the drug concentration in the composition, the more of the cellulosic polymer will be required to provide a crystallization-inhibiting effect. Generally, the crystallization inhibitor, if present, and the drug are present in a ratio of about 1:100 to about 1:1, preferably about 1:50 to about 1:1 and more preferably about 1:25 to about 1:1, by weight.

When certain poorly water-soluble drugs are formulated in dissolved or solubilized form in PEG, it has been found that impurities can be generated during storage. For example, in the case of a celecoxib solution composition in PEG-400, the impurities have been traced to reaction of the celecoxib not with PEG-400 itself but with a breakdown product of PEG-400. Without being bound by theory, it is believed that the breakdown product that reacts with celecoxib is ethylene oxide. Products of the reaction include addition compounds. It is contemplated that any drug compound having an aminosulfonyl functional group has a potential to react with a polyethylene glycol breakdown product in a similar way.

The problem of chemical instability of such a drug in a polyethylene glycol solvent, or indeed of any drug that can react with polyethylene glycol or a breakdown product thereof to form an addition compound, can be overcome by including a free radical-scavenging antioxidant in the solvent liquid.

Therefore, a composition optionally further comprises at least one pharmaceutically acceptable free radical-scavenging antioxidant. A free radical-scavenging antioxidant is to be contrasted with a "non-free radical-scavenging antioxidant", i.e., an antioxidant that does not possess free radical-scavenging properties. Non-limiting illustrative examples of suitable free radical-scavenging antioxidants include α-tocopherol (vitamin E), ascorbic acid (vitamin C) and salts thereof including sodium ascorbate and ascorbic acid palmitate, butylated hydroxyanisole (BHA), butylated hydroxytoluene (BHT), fumaric acid and salts thereof, hypophosphorous acid, malic acid, alkyl gallates, for example propyl gallate, octyl gallate and lauryl gallate, sodium thiosulfate, sodium sulfite, sodium bisulfite and sodium metabisulfite. Preferred free radical-scavenging antioxidants are alkyl gallates, vitamin E, BHA and BHT. More preferably the at least one free radical-scavenging antioxidant is propyl gallate.

One or more free radical-scavenging antioxidants are optionally present in compositions of the invention in a total amount effective to substantially reduce formation of an addition compound, typically in a total amount of about 0.01 % to about 5%, preferably about 0.01% to about 2.5%, and more preferably about 0.01% to about 1%, by weight of the composition.

A composition optionally comprises one or more pharmaceutically acceptable sweeteners. Non-limiting examples of suitable sweeteners include mannitol, propylene glycol, sodium saccharin, acesulfame K, neotame and aspartame. Alternatively or in addition, a viscous sweetener such as sorbitol solution, syrup (sucrose solution) or high-fructose corn syrup can be used and, in addition to sweetening effects, can also be useful to increase viscosity and to retard sedimentation. Use of sweeteners is especially advantageous in imbibable compositions of the invention, as these can be tasted by the subject prior to swallowing. An encapsulated composition does not typically interact with the organs of taste in the mouth and use of a sweetener is normally unnecessary.

A composition optionally comprises one or more pharmaceutically acceptable preservatives other than free radical-scavenging antioxidants. Non-limiting examples of suitable preservatives include benzalkonium chloride, benzethonium chloride, benzyl alcohol, chlorobutanol, phenol, phenylethyl alcohol, phenylmercuric nitrate, thimerosal, *etc*.

A composition optionally comprises one or more pharmaceutically acceptable wetting agents. Surfactants, hydrophilic polymers and certain clays can be useful as wetting agents to aid in dissolution and/or dispersion of a hydrophobic drug such as celecoxib. Non-limiting examples of suitable surfactants include benzalkonium chloride, benzethonium chloride, cetylpyridinium chloride, dioctyl sodium sulfosuccinate, nonoxynol 9, nonoxynol 10, octoxynol 9, poloxamers, polyoxyethylene (8) caprylic/capric mono- and diglycerides (*e.g.*, Labrasol^{™} of Gattefossé), polyoxyethylene (35) castor oil, polyoxyethylene (20) cetostearyl ether, polyoxyethylene (40) hydrogenated castor oil, polyoxyethylene (10) oleyl ether, polyoxyethylene (40) stearate, polysorbate 20, polysorbate 40, polysorbate 60, polysorbate 80 (*e.g.*, Tween^{™} 80 of ICI), propylene glycol laurate (*e.g.*, Lauroglycol^{™} of Gattefossé), sodium lauryl sulfate, sorbitan monolaurate, sorbitan monooleate, sorbitan monopalmitate, sorbitan monostearate, tyloxapol, and mixtures thereof.

Additionally, compositions optionally comprise one or more pharmaceutically acceptable buffering agents, flavoring agents, colorants, stabilizers and/or thickeners. Buffers can be used to control pH of a formulation and can thereby modulate drug solubility. Flavoring agents can enhance patient compliance by making the composition more palatable, particularly in the case of an imbibable composition, and colorants can provide a product with a more aesthetic and/or distinctive appearance. Non-limiting examples of suitable colorants include D&C Red No. 33, FD&C Red No. 3, FD&C Red No. 40, D&C Yellow No. 10, and C Yellow No. 6.

### Solution/suspension compositions

In one embodiment, the solvent liquid, depending on the particular components present therein, is suitable to maintain a first portion of drug in solution to provide a therapeutically effective rapid-onset dose while also maintaining a second portion of the drug undissolved but in suspension. The suspended portion typically provides less immediate release of the drug and so can extend the duration of therapeutic effect, although such extended duration is not a requirement of this embodiment of the invention.

Therefore, a composition is provided comprising a therapeutically effective amount of a poorly water-soluble drug, in part dissolved and in part dispersed in a pharmaceutically acceptable solvent liquid that comprises at least one solvent, at least one fatty acid and at least one organic amine. In this embodiment, part of the drug is in solution and part is in suspension. The composition further comprises a crystallization inhibitor as described above, the crystallization inhibitor being present in the solvent liquid and/or as a component of a capsule wall.

Preferably, the components of the solvent liquid are selected such that at least about 15% by weight of the drug is in dissolved or solubilized form in the solvent liquid. One way of modifying a solvent liquid to increase the amount of the poorly water soluble drug in suspension as opposed to solution is to add water in an amount necessary to give the required reduction in solubility of the drug in the solvent liquid.

Depending on the relative importance of rapid onset and sustained action for the indication for which the drug is being administered, the relative proportions of dissolved and suspended drug can be varied significantly. For example, for acute pain indications, about 50% of the drug can be in solution and about 50% of the drug can be dispersed in particulate form. Alternatively, for indications demanding longer acting therapeutic effectiveness, illustratively about 20% of the drug can be in solution and about 80% of the drug can be dispersed in particulate form.

The particulate form of the drug can be generated mechanically, for example by milling or grinding, or by precipitation from solution. Particles formed directly from such processes are described herein as "primary particles" and can agglomerate to form secondary aggregate particles. The term "particle size" as used herein refers to size, in the longest dimension, of primary particles, unless the context demands otherwise. Particle size is believed to be an important parameter affecting the clinical effectiveness of celecoxib and other drugs of low water solubility.

Particle size can be expressed as the percentage of total particles that have a diameter smaller than a given reference diameter. For example, a useful parameter is "D₉₀ particle size". By definition, in a batch of a drug that has a D₉₀ particle size of 60 µm, 90% of the particles, by volume, have a diameter less than 60 µm. For practical purposes a determination of D₉₀ based on 90% by weight rather than by volume is generally suitable.

Compositions preferably have a distribution of suspended drug particle sizes such that D₉₀ of the particles, in their longest dimension, is about 0.5 µm to about 200 µm, preferably about 0.5 µm to about 75 µm, and more preferably about 0.5 µm to about 25 µm. For example, where the drug is celecoxib, a decrease in particle size in accordance with this embodiment of the invention generally improves drug bioavailability. In addition or alternatively, suspended celecoxib particles in a composition of the invention preferably have a mean particle size less than about 10 µm, more preferably about 0.1 µm to about 10 µm, and most preferably about 0.5 µm to about 5 µm, for example about 1 µm.

Compositions can optionally comprise additional excipients such as crystallization inhibitors, dispersants, co-solvents, sweeteners, preservatives, emulsifying agents, *etc.*, as described above. Further, compositions of this embodiment can be formulated either in imbibable or discrete dosage form.

Additionally, certain excipients such as suspending agents, thickening agents and flocculating agents can be particularly useful where suspended drug particles are desired, for example in solution/suspension compositions. Through selection and combination of excipients, solution/suspension compositions can be provided exhibiting improved performance with respect to drug concentration, physical stability, efficacy, flavor, and overall patient compliance.

Solution/suspension compositions optionally comprise one or more pharmaceutically acceptable suspending agents. Suspending agents are used to impart increased viscosity and retard sedimentation. Suspending agents are of various classes including cellulose derivatives, clays, natural gums, synthetic gums and miscellaneous agents. Non-limiting examples of suspending agents that can be used in compositions of the present invention include acacia, agar, alginic acid, aluminum monostearate, attapulgite, bentonite, carboxymethylcellulose calcium, carboxymethylcellulose sodium, carrageenan, carbomer, for example carbomer 910, dextrin, ethylmethylcellulose, gelatin, guar gum, HPMC, methylcellulose, ethylcellulose, ethylhydroxyethylcellulose, hydroxyethylcellulose, hydroxypropylcellulose, kaolin, magnesium aluminum silicate, microcrystalline cellulose, microcrystalline cellulose with carboxymethylcellulose sodium, powdered cellulose, silica gel, colloidal silicon dioxide, locust bean gum, pectin, sodium alginate, propylene glycol alginate, tamarind gum, tragacanth, xanthan gum, povidone, veegum, glycyrrhizin, pregelatinized starch, sodium starch glycolate and mixtures thereof.

In certain circumstances, it can be desirable to use flocculating agents in solution/suspension compositions of the invention. Flocculating agents enable particles to link together in loose aggregates or flocs and include surfactants, hydrophilic polymers, clays and electrolytes. Non-limiting examples of suitable flocculating agents include sodium lauryl sulfate, docusate sodium, benzalkonium chloride, cetylpyridinium chloride, polysorbate 80, sorbitan monolaurate, carboxymethylcellulose sodium, xanthan gum, tragacanth, methylcellulose, PEG, magnesium aluminum silicate, attapulgite, bentonite, potassium dihydrogen phosphate, aluminum chloride, sodium chloride and mixtures thereof.

### Discrete dosage forms

It has been found that the demands of a rapid-onset formulation are met surprisingly well by a preparation containing a solution or solution/suspension of the present invention encapsulated as a discrete dosage unit article. Therefore, another embodiment of the present invention is a concentrated composition, either a solution or solution/suspension, wherein the composition is formulated as one or more discrete dose units, for example soft or hard capsules.

Any suitable encapsulation material, for example gelatin or HPMC, can be used. As indicated hereinabove, a turbidity-decreasing polymer can be an advantageous material for use in the capsule wall because it can act as a crystallization inhibitor upon exposure of the composition to gastrointestinal fluid. A polymer component such as HPMC is "present in the capsule wall" or is a "capsule wall component" as described herein if the polymer is (a) dispersed or mixed together with any other capsule wall component(s), (b) the only capsule wall component, or (c) present as a coating on the outside or inside of the capsule wall.

In a presently preferred embodiment, a cellulosic polymer having methoxyl and/or hydroxypropoxyl substitution as described hereinabove, preferably HPMC, is present in the capsule wall in a total amount of about 5% to substantially 100%, and preferably about 15% to substantially 100%, by weight of the wall. In addition to one or more such cellulosic polymers, a suitable capsule wall can comprise any additional component useful in the art such as gelatin, starch, carrageenan, sodium alginate, plasticizers, potassium chloride, coloring agents, etc. A suitable capsule herein may have a hard or soft wall.

The crystallization inhibitor is preferably present in the wall in a total amount sufficient to substantially inhibit drug crystallization and/or precipitation upon dissolution, dilution and/or degradation of the composition in SGF. For practical purposes, whether an amount of crystallization inhibitor present in the wall of a given test composition is sufficient to substantially inhibit drug crystallization and/or precipitation can be determined according to Test IV, which can also be used to determine whether a particular polymer component is useful as a crystallization inhibitor when present in the capsule wall of a particular composition of the invention.

### Test IV:

A. A volume of a solution or solution/suspension as described herein above is enclosed in a capsule comprising a test polymer to form a test composition, and is placed in a volume of SGF to form a mixtures having a fixed ratio of about 1 g to about 2 g of the composition per 100 ml of SGF.
B. The mixture is maintained at a constant temperature of about 37°C and is stirred using type II paddles (USP 24) at a rate of 75 rpm for a period of 4 hours.
C. At one or more time-points after at least about 15 minutes of stirring but before about 4 hours of stirring, an aliquot of the mixture is drawn and filtered, for example through a non-sterile Acrodisc^{™} syringe filter with a 0.8 µm Versapor^{™} membrane.
D. Filtrate is collected in a vessel.
E. Drug concentration in the filtrate is measured using high performance liquid chromatography (HPLC).
F. The test is repeated identically with a comparative composition comprising a solution or solution/suspension that is substantially similar to the solution or solution/suspension used in Step A but which is enclosed in a capsule comprising no crystallization inhibitor (i.e.. comprises no polymer or, if a polymer is present, it is a polymer such as gelatin which does not inhibit crystallization and/or precipitation). The polymer component is replaced in the capsule enclosing the comparative composition with gelatin.
G. If the drug concentration in the filtrate resulting from the test composition is greater than that in the filtrate resulting from the comparative composition, the polymer component present in the capsule wall of the test composition is deemed to be present in an amount sufficient to substantially inhibit crystallization and/or precipitation of the drug in SGF.

Where a crystallization-inhibiting cellulosic polymer is present as a capsule wall component, the solution or solution/suspension contained therein can additionally, but optionally, comprise a further amount of such a cellulosic polymer.

Preferably, one to about six, more preferably one to about four, and still more preferably one or two of such discrete dosage units per day provides a therapeutically effective dose of the drug.

Compositions are preferably formulated such that each discrete dosage unit contains about 0.3 ml to about 1.5 ml, more preferably about 0.3 ml to about 1 ml, for example about 0.8 ml or about 0.9 ml, of solution or solution/suspension.

Concentrated solutions or solutions/suspensions can be encapsulated by any method known in the art including the plate process, vacuum process, or the rotary die process. See, for example, Ansel et al. (1995) in Pharmaceutical Dosage Forms and Drug Delivery Systems, 6th ed., Williams & Wilkins, Baltimore, MD, pp. 176-182. By the rotary die process, liquid encapsulation material, for example gelatin, flowing from an overhead tank is formed into two continuous ribbons by a rotary die machine and brought together by twin rotating dies. Simultaneously, metered fill material is injected between ribbons at the same moment that the dies form pockets of the ribbons. These pockets of fill-containing encapsulation material are then sealed by pressure and heat, and the capsules are served from the machine.

Soft capsules can be manufactured in different shapes including round, oval, oblong, and tube-shape, among others. Additionally, by using two different ribbon colors, two-tone capsules can be produced.

Capsules that comprise HPMC are known in the art and can be prepared, sealed and/or coated, by way of non-limiting illustration, according to processes disclosed in the patents and publications listed below, each of which is individually incorporated herein by reference.
United States Patent No. 4,250,997 to Bodenmann et al*.*
United States Patent No. 5,264,223 to Yamamoto et al*.*
United States Patent No. 5,756,123 to Yamamoto et al*.*
International Patent Publication No. WO 96/05812.
International Patent Publication No. WO 97/35537.
International Patent Publication No. WO 00/18377.
International Patent Publication No. WO 00/27367.
International Patent Publication No. WO 00/28976.
International Patent Publication No. WO 01/03676.
European Patent Application No. 0211 079.
European Patent Application No. 0 919 228.
European Patent Application No. 1 029 539.
Non-limiting illustrative examples of suitable HPMC-comprising capsules include XGel^{™} capsules of Bioprogress and Qualicaps^{™} of Shionogi.

### Imbibable dosage forms

A concentrated composition, either a concentrated solution or a concentrated solution/suspension can be directly imbibed or diluted with inert diluents and/or other carriers and imbibed; such compositions, whether diluted or not, are referred to for convenience herein as "imbibable compositions". Imbibable compositions can be prepared by any suitable method of pharmacy that includes the steps of bringing into association the drug of low water solubility, illustratively celecoxib, and the components of the solvent liquid. As there is no capsule wall in this embodiment, if it is desired to include a crystallization inhibitor it must be present in the solvent liquid. Where the drug is celecoxib, compositions of this embodiment preferably contain about 40 mg/ml to about 750 mg/ml, more preferably about 50 mg/ml to about 500 mg/ml, still more preferably about 50 mg/ml to about 350 mg/ml, and most preferably, about 100 mg/ml to about 300 mg/ml, for example about 200 mg/ml, of celecoxib.

Solutions or solution/suspensions are provided that are required to be diluted to provide a dilution suitable for direct, imbibable administration. Solutions or solution/suspensions of the present invention are added, in a therapeutically effective dosage amount, to about 1 ml to about 20 ml of an inert liquid. Preferably solutions or solution/suspensions of the present invention are added to about 2 ml to about 15 ml, and more preferably to about 5 ml to about 10 ml, of inert liquid. The term "inert liquid" as used herein refers to pharmaceutically acceptable, preferably palatable liquid carriers. Such carriers are typically aqueous. Examples include water, fruit juices, carbonated beverages, etc.

### Method of preparing a composition

A composition can be prepared according to any suitable admixing process. Illustratively, such a composition can be prepared by dissolving, in a first vessel, the at least one pharmaceutically acceptable amine, preferably an organic amine, in a solvent (*e.g.*, water) with heat (*e.g.*, about 50°C to about 60°C) to form a heated mixture. Preferably the weight ratio of organic amine to solvent in the heated mixture is about 1:5 to about 5:1 and more preferably about 1:2 to about 2:1, for example about 1:1. In a second vessel, the at least one pharmaceutically acceptable fatty acid, any additional solvents (*e.g.*, the solvent for the fatty acid and/or for the drug of low water solubility), the at least one pharmaceutically acceptable drug of low water solubility in solid, dissolved and/or solubilized form, and any other desired excipients are admixed together to form a secondary mixture. The heated mixture and the secondary mixture are then admixed together. Without being bound by theory, it is believed that the heated mixture must be maintained at an elevated temperature until it is admixed with the secondary mixture in order to prevent precipitation and/or crystallization of the organic amine from solution. It is also believed, without being held to a particular theory, that if the organic amine does precipitate out of solution prior to admixing with the secondary mixture, a suitable fatty acid/organic amine pair will not be formed and the final formulation is less likely to be finely self-emulsifiable in simulated gastric fluid.

Unfortunately, when performed on a larger scale, for example commercial scale, it is expensive and inefficient to maintain the organic amine in solution using heat, for example where transfer of the heated mixture through a large pipe for further processing is desired or required. In such a case, the transfer pipe would have to be internally heated and/or thermally jacketed in order to prevent crystallization of the organic amine. Such heating is expensive, inefficient, and potentially deleterious to other ingredients in the formulation.

We have now discovered a process for preparing a composition of the invention which is particularly advantageous for large scale preparation and which overcomes the problem of organic amine precipitation without the need for continuous and expensive heating of the organic amine mixture. A process of this embodiment comprises the steps of:
(a) providing at least one pharmaceutically acceptable fatty acid and at least one pharmaceutically acceptable amine, preferably an organic amine;
(b) providing a pharmaceutically acceptable solvent for the at least one fatty acid and a pharmaceutically acceptable solvent for the at least one organic amine;
(c) admixing together with the at least one fatty acid the solvent for the at least one fatty acid and the solvent for the at least one organic amine to form a pre-mix in which the fatty acid is substantially completely dissolved;
(d) admixing together with the pre-mix the at least one organic amine to form a mixture in which the organic amine is substantially completely dissolved; and
(e) admixing together with the mixture the drug of low water solubility in solid, dissolved and/or solubilized form to form a pharmaceutical composition. It is preferable that the drug of low water solubility is in dissolved and/or solubilized form prior to admixing the drug with the mixture of step (d). Illustratively, one or more solvents can be used to solubilize the drug prior to use in step (e).

Preferably, step (c) is performed prior to step (d); however, if desired, step (c) and step (d) can be performed simultaneously. The term "admixing" in the present context means adding together of two or more components with agitation, for example with stirring using a magnetic stir bar. If desired, heat can be applied at any step of the process, for example, to facilitate dissolution of the fatty acid and the organic amine. Illustratively, steps (c) and (d) can be performed at a temperature of about 40°C to about 60°C, and more preferably at a temperature of about 45°C to about 55°C.

However, according to a process of this embodiment, after the fatty acid and organic amine are in solution, heat is not required to maintain such a solution as the mixture resulting from step (d) is physically stable at room temperature. Therefore, step (e) is preferably performed at a temperature of about 15 °C to about 30°C, and more preferably about 20°C to about 25°C.

Preferably, the solvent(s) used in a process of this embodiment are selected so as to be not only suitable (by type and amount) to dissolve and/or solubilize the fatty acid and organic amine, individually and/or collectively, but also to dissolve any other would-be precipitates that may tend to form during execution of the process.

Surprisingly, we have now discovered that a process of this embodiment leads to a mixture resulting from step (d) which is physically stable (*i.e.*, exhibiting substantially no precipitation or crystallization of organic amine or other components) even when maintained at room temperature. As indicated above, this surprising advantage makes a process according to this embodiment particularly useful for large-scale preparation, for example where a large volume of such a mixture is to be transferred through a pipe for subsequent admixture with other desired ingredients.

In a particularly preferred process of this embodiment, where the at least one pharmaceutically acceptable organic amine is tromethamine, the pharmaceutically acceptable solvent for the at least one organic amine is water; and where the at least one fatty acid is oleic acid, the pharmaceutically acceptable solvent for the at least one fatty acid is ethanol.

### Utility of compositions that comprise a selective COX-2 inhibitors drug

Compositions comprise an aminosulfonyl-comprising selective COX-2 inhibitory drug of low water solubility. Compositions of this embodiment are useful in treatment and prevention of a very wide range of disorders mediated by COX-2, including but not restricted to disorders characterized by inflammation, pain and/or fever. Such compositions are especially useful as anti-inflammatory agents, such as in treatment of arthritis, with the additional benefit of having significantly less harmful side effects than compositions of conventional nonsteroidal anti-inflammatory drugs (NSAIDs) that lack selectivity for COX-2 over COX-1. In particular, such compositions have reduced potential for gastrointestinal toxicity and gastrointestinal irritation including upper gastrointestinal ulceration and bleeding, reduced potential for renal side effects such as reduction in renal function leading to fluid retention and exacerbation of hypertension, reduced effect on bleeding times including inhibition of platelet junction, and possibly a lessened ability to induce asthma attacks in aspirin-sensitive asthmatic subjects, by comparison with compositions of conventional NSAIDs. Thus compositions of the invention comprising a selective COX-2 inhibitory drug are particularly useful as an alternative to conventional NSAIDs where such NSAIDs are contraindicated, for example in patients with peptic ulcers, gastritis, regional enteritis, ulcerative colitis, diverticulitis or with a recurrent history of gastrointestinal lesions; gastrointestinal bleeding, coagulation disorders including anemia such as hypoprothrombinemia, hemophilia or other bleeding problems; kidney disease; or in patients prior to surgery or patients taking anticoagulants.

Such compositions are useful to treat a variety of arthritic disorders, including but not limited to rheumatoid arthritis, spondyloarthropathies, gouty arthritis, osteoarthritis, systemic lupus erythematosus and juvenile arthritis.

Such compositions are also useful in treatment of asthma, bronchitis, menstrual cramps, preterm labor, tendinitis, bursitis, allergic neuritis, cytomegalovirus infectivity, apoptosis including HIV-induced apoptosis, lumbago, liver disease including hepatitis, skin-related conditions such as psoriasis, eczema, acne, burns, dermatitis and ultraviolet radiation damage including sunburn, and post-operative inflammation including that following ophthalmic surgery such as cataract surgery or refractive surgery.

Such compositions are useful to treat gastrointestinal conditions such as inflammatory bowel disease, Crohn's disease, gastritis, irritable bowel syndrome and ulcerative colitis.

Such compositions are useful in treating inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of ophthalmic diseases, such as retinitis, conjunctivitis, retinopathies, uveitis, ocular photophobia, and of acute injury to the eye tissue.

Such compositions are useful in treatment of pulmonary inflammation, such as that associated with viral infections and cystic fibrosis, and in bone resorption such as that associated with osteoporosis.

Such compositions are useful for treatment of certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, burns, and trauma following surgical and dental procedures.

Such compositions are useful for treating and preventing inflammation-related cardiovascular disorders, including vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful in treatment of angiogenesis-related disorders in a subject, for example to inhibit tumor angiogenesis. Such compositions are useful in treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; and disorders of the female reproductive system such as endometriosis.

Such compositions are useful in prevention and treatment of benign and malignant tumors and neoplasia including cancer, such as colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer, skin cancer such as squamous cell and basal cell cancers, prostate cancer, renal cell carcinoma, and other known cancers that effect epithelial cells throughout the body. Neoplasias for which compositions of the invention are contemplated to be particularly useful are gastrointestinal cancer, Barrett's esophagus, liver cancer, bladder cancer, pancreatic cancer, ovarian cancer, prostate cancer, cervical cancer, lung cancer, breast cancer and skin cancer. Such compositions can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in patients at risk of FAP.

Such compositions inhibit prostanoid-induced smooth muscle contraction by inhibiting synthesis of contractile prostanoids and hence can be of use in treatment of dysmenorrhea, premature labor, asthma and eosinophil-related disorders. They also can be of use for decreasing bone loss particularly in postmenopausal women (*i.e*., treatment of osteoporosis), and for treatment of glaucoma.

Because of the rapid onset of therapeutic effect that can be exhibited by compositions of the invention, these compositions have particular advantages over prior formulations for treatment of acute COX-2 mediated disorders, especially for relief of pain, for example in headache, including sinus headache and migraine.

Preferred uses for compositions of the present invention are for treatment of rheumatoid arthritis and osteoarthritis, for pain management generally (particularly post-oral surgery pain, post-general surgery pain, post-orthopedic surgery pain, and acute flares of osteoarthritis), for prevention and treatment of headache and migraine, for treatment of Alzheimer's disease, and for colon cancer chemoprevention.

For treatment of rheumatoid arthritis or osteoarthritis, such compositions of the invention can be used to provide a daily dosage of celecoxib of about 50 mg to about 1000 mg, preferably about 100 mg to about 600 mg, more preferably about 150 mg to about 500 mg, still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in a composition of the invention. The daily dose can be administered in one to about four doses per day, preferably one or two doses per day.

For treatment of Alzheimer's disease or cancer, such compositions of the invention can be used to provide a daily dosage of celecoxib of about 50 mg to about 1000 mg, preferably about 100 mg to about 800 mg, more preferably about 150 mg to about 600 mg, and still more preferably about 175 mg to about 400 mg, for example about 400 mg. A daily dose of about 0.7 to about 13 mg/kg body weight, preferably about 1.3 to about 10.7 mg/kg body weight, more preferably about 2 to about 8 mg/kg body weight, and still more preferably about 2.3 to about 5.3 mg/kg body weight, for example about 5.3 mg/kg body weight, is generally appropriate when administered in as composition of the invention. The daily dose can be administered in one to about four doses per day, preferably one or two doses per day.

For pain management generally and specifically for treatment and prevention of headache and migraine, such compositions of the invention can be used to provide a daily dosage of celecoxib of about 50 mg to about 1000 mg, preferably about 100 mg to about 600 mg, more preferably about 150 mg to about 500 mg, and still more preferably about 175 mg to about 400 mg, for example about 200 mg. A daily dose of celecoxib of about 0.7 to about 13 mg/kg body weight, preferably about 1.3 to about 8 mg/kg body weight, more preferably about 2 to about 6.7 mg/kg body weight, and still more preferably about 2.3 to about 5.3 mg/kg body weight, for example about 2.7 mg/kg body weight, is generally appropriate when administered in a composition of the invention. The daily dose can be administered in one to about four doses per day. Administration at a rate of one 50 mg dose unit four times a day, one 100 mg dose unit or two 50 mg dose units twice a day or one 200 mg dose unit, two 100 mg dose units or four 50 mg dose units once a day is preferred.

For selective COX-2 inhibitory drugs other than celecoxib, appropriate doses can be selected by reference to the patent literature cited hereinabove.

Besides being useful for human treatment, such compositions of the invention are useful for veterinary treatment of companion animals, exotic animals, farm animals, and the like, particularly mammals. More particularly, such compositions of the invention are useful for treatment of COX-2 mediated disorders in horses, dogs and cats.

A therapeutic method of treating a condition or disorder where treatment with a COX-2 inhibitory drug is indicated, the method comprising oral administration of a composition of the invention to a subject in need thereof. The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the subject and the nature and severity of the disorder. Thus, the dosage regimen actually employed can vary widely and can therefore deviate from the preferred dosage regimens set forth above.

Initial treatment can begin with a dose regimen as indicated above. Treatment is generally continued as necessary over a period of several weeks to several months or years until the condition or disorder has been controlled or eliminated. Subjects undergoing treatment with a composition of the invention can be routinely monitored by any of the methods well known in the art to determine effectiveness of therapy. Continuous analysis of data from such monitoring permits modification of the treatment regimen during therapy so that optimally effective doses are administered at any point in time, and so that the duration of treatment can be determined. In this way, the treatment regimen and dosing schedule can be rationally modified over the course of therapy so that the lowest amount of the composition exhibiting satisfactory effectiveness is administered, and so that administration is continued only for so long as is necessary to successfully treat the condition or disorder.

Compositions can be used in combination therapies with opioids and other analgesics, including narcotic analgesics, Mu receptor antagonists, Kappa receptor antagonists, non-narcotic (i.e. non-addictive) analgesics, monoamine uptake inhibitors, adenosine regulating agents, cannabinoid derivatives, Substance P antagonists, neurokinin-1 receptor antagonists and sodium channel blockers, among others. Preferred combination therapies comprise use of a composition of the invention with one or more compounds selected from aceclofenac, acemetacin, *e*-acetamidocaproic acid, acetaminophen, acetaminosalol, acetanilide, acetylsalicylic acid (aspirin), *S*-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, *p*-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil lonazolac, lornoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide *o*-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, virninol, xenbucin, ximoprofen, zaltoprofen and zomepirac (see The Merck Index, 12th Edition (1996), Therapeutic Category and Biological Activity Index, lists therein headed "Analgesic", "Anti-inflammatory" and "Antipyretic").

Particularly preferred combination therapies comprise use of a composition of this embodiment with an opioid compound, more particularly where the opioid compound is codeine, meperidine, morphine or a derivative thereof

The compound to be administered in combination with a selective COX-2 inhibitory drug can be formulated separately from the drug or co-formulated with the drug in a composition of the invention. Where a selective COX-2 inhibitory drug is co-formulated with a second drug, for example an opioid drug, the second drug can be formulated in immediate-release, rapid-onset, sustained-release or dual-release form.

In an embodiment of the invention, particularly where the COX-2 mediated condition is headache or migraine, the present selective COX-2 inhibitory drug composition is administered in combination therapy with a vasomodulator, preferably a xanthine derivative having vasomodulatory effect, more preferably an alkylxanthine compound.

Combination therapies wherein an alkylxanthine compound is co-administered with a selective COX-2 inhibitory drug composition as provided herein are embraced by the present embodiment of the invention whether or not the alkylxanthine is a vasomodulator and whether or not the therapeutic effectiveness of the combination is to any degree attributable to a vasomodulatory effect. The term "alkylxanthine" herein embraces xanthine derivatives having one or more C₁₋₄ alkyl, preferably methyl, substituents, and pharmaceutically acceptable salts of such xanthine derivatives. Dimethylxanthines and trimethylxanthines, including caffeine, theobromine and theophylline, are especially preferred. Most preferably, the alkylxanthine compound is caffeine.

The total and relative dosage amounts of the selective COX-2 inhibitory drug and of the vasomodulator or alkylxanthine are selected to be therapeutically and/or prophylactically effective for relief of pain associated with the headache or migraine. Suitable dosage amounts will depend on the particular selective COX-2 inhibitory drug and the particular vasomodulator or alkylxanthine selected. For example, in a combination therapy with celecoxib and caffeine, typically the celecoxib will be administered in a daily dosage amount of about 50 mg to about 1000 mg, preferably about 100 mg to about 600 mg, and the caffeine in a daily dosage amount of about 1 mg to about 500 mg, preferably about 10 mg to about 400 mg, more preferably about 20 mg to about 300 mg.

The vasomodulator or alkylxanthine component of the combination therapy can be administered in any suitable dosage form by any suitable route, preferably orally. The vasomodulator or alkylxanthine can optionally be coformulated with the selective COX-2 inhibitory drug in a single oral dosage form. Thus a solution or solution/suspension formulation of the invention optionally comprises both an aminosulfonyl-comprising selective COX-2 inhibitory drug and a vasomodulator or alkylxanthine such as caffeine, in total and relative amounts consistent with the dosage amounts set out hereinabove.

The phrase "in total and relative amounts effective to relieve pain", with respect to amounts of a selective COX-2 inhibitory drug and a vasomodulator or alkylxanthine in a composition of the present embodiment, means that these amounts are such that (a) together these components are effective to relieve pain, and (b) each component is or would be capable of contribution to a pain-relieving effect if the other component is or were not present in so great an amount as to obviate such contribution.

### EXAMPLES

### Reference Example 1

Six celecoxib solution formulations SF-1 to SF-6 were prepared as shown in Table 1.

**Table 1. Composition (mg) of celecoxib solution formulations SF-1 to SF-6**

| **Component** | **SF-1** | **SF-2** | **SF-3** | **SF-4** | **SGF-5** | **SF-6** |
|---|---|---|---|---|---|---|
| Celecoxib | 250 | 250 | 250 | 250 | 250 | 250 |
| Tagat^{™} TO | 400 | 400 | 400 | 400 | 400 | 400 |
| Transcutol^{™} | 230 | 230 | 230 | 230 | 230 | 230 |
| Oleic acid (OA) | 90 | 90 | 90 | 90 | 90 | 90 |
| Dimethylethanolamine (DA) | -- | 7 | 12.8 | 20 | 40 | 82 |
| Mole ratio OA:DA | 1:0 | 1:0.23 | 1:0.45 | 1:0.7 | 1:1.4 | 1:2.9 |

### Reference Example 2

An *in vitro* assay was performed to determine selt-emulsification properties of celecoxib solution formulations SF-1 to SF-6 of Example 1 as follows:
(a) 400 µl of a solution formulation was placed into a screw-top, side-arm vessel containing 20 ml SGF, maintained at 37°C throughout the test, to form a test liquid;
(b) the test liquid was mildly agitated at 75 rpm for 2 minutes using an orbital shaker;
(c) a 5-50 µl aliquot of the test liquid was withdrawn through the side-arm using a pipette and was discharged from the pipette into a sampling vessel;
(d) a pump (model RHOCKC-LF; Fluid Metering Inc.; Syosset, NY) was used to pull the test liquid from the sampling vessel through a combination scattering/obscuration sensor (LE400-0.5; Particle Sizing Systems; Santa Barbara, CA) at the rate of 1 ml/minute for a period of 1 minute;
(e) emulsion particles were counted individually by light scattering between 0.5 and 1 µm and by light obscuration in the size range above 1 µm using the vendor's software (Version 1.59);
(f) a plot was prepared as number (unweighted) or volume (weighted) of emulsion particle counts versus diameter;
(g) integration of the plot, accounting for all dilutions, was performed to estimate total mass of material present in the mixture that was large enough to be detected by the sensor.

The resulting data, shown in Table 2, indicate that, at a given level of oleic acid, the presence of a sufficient amount of an organic amine in a composition of the invention renders the solution formulation finely self-emulsifiable in simulated gastric fluid.

**Table 2. Self emulsifying properties of formulations SF-1 to SF-6 in SGF**

| **Formulation** | **Volume % of particles ≥1 µm** | **Qualitative emulsion description** |
|---|---|---|
| SF-1 | 50 | poorly dispersed large oily particles |
| SF-2 | 65 | poorly dispersed large oily particles |
| SF-3 | 46 | substantial amount of large oily particles |
| SF-4 | 31 | rapid dispersion to submicron particles with a low fraction of large particles |
| SF-5 | 3 | rapid dispersion to submicron particles with a low fraction of large particles |
| SF-6 | 2 | rapid dispersion to submicron particles |

### Reference Example 3

Six celecoxib solution formulations SF-7 to SF-12 were prepared as shown in Table 3.

**Table 3. Composition (mg) of celecoxib solution formulations SF-7 to SF-12**

| **Component** | **SF-7** | **SF-8** | **SF-9** | **SF-10** | **SF-11** | **SF-12** |
|---|---|---|---|---|---|---|
| Celecoxib | 200 | 200 | 200 | 200 | 200 | 200 |
| PEG-400 | 440 | 440 | 440 | 440 | 440 | 440 |
| Tween^{™} 80 | 250 | 250 | 250 | 250 | 250 | 250 |
| Oleic acid (OA) | 90 | 90 | 90 | 90 | 90 | 90 |
| Ethanolamine (EA) | -- | 4.9 | 9.9 | 14.8 | 19 | 29 |
| Mole ratio OA:EA | 1:0 | 1:0.25 | 1:0.5 | 1:0.75 | 1:1 | 1:1.5 |

### Reference Example 4

An *in vitro* assay, as describe in Example 2, was performed on solution formulations SF-7 to SF-12. Data are shown in Table 4.

**Table 4. Self-emulsifying properties of formulations SF-7 to SF-12 in SGF**

| **Formulation** | **Volume % of particles ≥1 µm** | **Qualitative emulsion description** |
|---|---|---|
| SF-7 | 100 | essentially undispersed (some cloudiness); most material on bottom of vessel |
| SF-8 | 100 | largely undispersed (some cloudiness) |
| SF-9 | 17 | did not disperse immediately; within minutes the dilution medium was white, very cloudy |
| SF-10 | 2.7 | dispersed fairly well; within minutes the dilution medium was white, opaque |
| SF-11 | 5 | dispersed fairly well; within minutes the dilution medium was white, opaque. |
| SF-12 | 0.05 | very good dispersing qualities; rapid formation of white, opaque dispersion |

These data indicate that, at a given level of oleic acid, the presence of a sufficient amount of an organic amine in a composition of the invention renders the solution formulation finely self-emulsifiable in simulated gastric fluid.

### Reference Example 5

A celecoxib solution formulation, SF-13, was prepared as shown in Table 5.

**Table 5. Composition (mg/g) of celecoxib solution formulation SF-13**

| **Component** | **SF-13** |
|---|---|
| Celecoxib | 200 |
| Water USP | 26 |
| HPMC (E5) | 38 |
| Ethanol | 113 |
| PEG-400 | 271 |
| Polyvinylpyrrolidone | 47 |
| Polysorbate 80 | 217 |
| Tromethamine | 26 |
| Oleic acid | 61 |
| Propyl gallate NF | 1 |
| Total | 1000 |

One gram of SF-13 was individually placed into each of several hard gelatin capsules (Capsugel) to form test composition 1.

### Reference Example 6

A celecoxib suspension formulation was prepared for comparative purposes as follows:
(a) 5.0 g Tween^{™} 80 (polysorbate 80) was placed in a volumetric flask;
(b) ethanol was added (to 100 ml) to form a mixture and the mixture was swirled to form a uniform solution;
(c) 5 ml of the uniform solution was transferred to a fresh 100 ml bottle containing 200 mg celecoxib to form a pre-mix;
(d) 75 ml apple juice was added to the premix to form an intermediate celecoxib suspension; and
(e) the intermediate celecoxib suspension was left to stand for 5 minutes, and was then shaken to form a celecoxib suspension.

Bioavailability parameters resulting from administration of test composition 1 of Example 5, in comparison with the comparative celecoxib suspension composition of Example 6 and with a commercial celecoxib (Celebrex® of Pharmacia) 200 mg capsule, to human subjects were evaluated in a 24-subject, randomized, four period, balanced, crossover study. A fourth composition, not relevant to the present invention, was also included in the study but is not reported here. Study duration was approximately 15 days and subjects were randomly given one of each of the four dosage forms on days 1, 5, 9 and 12; administration of each dose was preceded by an 8 hour fasting period and was accompanied by 180 ml of water. Plasma blood levels for each subject were measured at pre-dose and at 15, 30, 45 minutes and 1, 1.5, 2, 3, 4, 6, 8,12 and 24 hours after dosage administration. Cₘₐₓ and AUC were calculated from the data in accordance with standard procedure in the art. As shown in Table 6, ingestion of test composition 1 resulted in a Cₘₐₓ more than 2.5 times greater than resulted from ingestion of the comparative celecoxib suspension or the commercial celecoxib capsule. Ingestion of test composition 1 also resulted in an AUC 43% greater than, and a Tₘₐₓ substantially similar to, that resulting from ingestion of the comparative celecoxib suspension.

**Table 6. In vivo bioavailability of celecoxib in human subjects**

| **Parameter** | **Commercial capsule** | **Comparative suspension** | **Test composition 1** |
|---|---|---|---|
| Cₘₐₓ (ng/ml) | 621 | 804 | 2061 |
| Tₘₐₓ(hr) | 2.15 | 0.97 | 1.03 |
| AUC (ng/ml)*hr | 5060 | 4892 | 7593 |

### Reference Example 7

Two celecoxib solution formulations, SF-14 and SF-15, were prepared having compositions shown in Table 7.

**Table 7. Composition (mg) of celecoxib solution formulations SF-14 - SF-15 and placebo solution formulations P-2 and P-3**

| **Component** | **SF-14** | **SF-15** | **P-2** | **P-3** |
|---|---|---|---|---|
| Celecoxib | 100 | 200 | - | - |
| Water USP | 13 | 26 | 15.1 | 30.2 |
| HPMC (ES) | 19 | 38 | 22.1 | 44.2 |
| Ethanol | 56.5 | 113 | 65.7 | 131.4 |
| PEG 400 | 135.5 | 271 | 157.5 | 315 |
| PVP | 23.5 | 47 | 27.3 | 54.6 |
| Polysorbate 80 | 108.5 | 217 | 126.1 | 252.3 |
| Tromethamine | 13 | 26 | 15.1 | 30.2 |
| Oleic acid | 30.5 | 61 | 35.5 | 70.9 |
| Propyl gallate NF | 0.5 | 1 | 0.6 | 1.2 |
| Total | 500 | 1000 | 465 | 930 |

Amounts of 500 mg and 1000 mg of solution formulations SF-12 and SF-13 respectively were individually placed into each of several soft gelatin capsules to form Test Compositions 2 (100 mg celecoxib) and 3 (200 mg celecoxib), respectively. Test Composition 4 consisted of two capsules of Test Composition 3 resulting in a 400 mg celecoxib dose. Placebo solution formulations P-2 and P-3 were filled into soft capsules corresponding in size with those containing solution formulations SF-12 and SF-13, respectively, to form Placebo Composition 2 and Placebo Composition 3.

A randomized, double-blind, active and placebo controlled, single-dose parallel group study was performed in order to assess the analgesic efficacy of Test Compositions 2, 3 and 4 in comparison with appropriate and visually matching placebo, in a human post-oral surgery pain model.

Post-surgical patients (after extraction of two or more impacted third molars requiring bone removal) who reported moderate or severe post-oral surgery pain on a categorical pain scale (CPS; 0 = no pain, 1 = mild pain, 2 = moderate pain, and 3 = severe pain), and a baseline pain intensity ≥50 mm on a visual analog scale (VAS; whereby patient locates a sliding bar representing his or her level of pain on a 100 mm horizontal scale with the left edge (0 mm) marked "no pain" and the right edge (100 mm) marked "worst pain") within 6 hours after completion of surgery were selected and randomized for study.

Each patient was randomized to one of four treatment groups (approximately 55 per group) and, 6 hours after completion of surgery, received the study medication assigned to his or her group from both Bottle A and Bottle B as shown in the medication schedule found in Table 8. Two additional compositions, not illustrative of the present invention, were also included in the study but are not reported here.

**Table 8. Schedule of study medication given to patients in treatment groups 1-4**

| **Treatment Group** | **Bottle A (1 capsule)** | **Bottle B (2 capsules)** |
|---|---|---|
| **1. (Placebo)** | 1 x Placebo Composition 2 | 2 x Placebo Composition 3 |
| **2. (Test composition 2)** | 1 x Test Composition 2 | 2 x Placebo Composition 3 |
| **3. (Test composition 3)** | 1 x Placebo Composition 2 | 1 x Placebo Composition 3 and 1 x Test Composition 3 |
| **4. (Test composition 4)** | 1 x Placebo Composition 2 | 2 x Test Composition 3 |

Pain was assessed at baseline (0 hour), 0.25, 0.50. 0.75, 1.0, 1.25, 1.50, 1.75, 2, 3, 4, 5, 6, 7, 8, 9, 10, 11, 12, 16, and 24 hours after administration of study medication. Each patient individually determined and recorded time to perceptible pain relief and time to meaningful pain relief, using two stopwatches.

Time to onset of analgesia was then calculated for each patient by performing a time-to-event analysis combining data from patient's stopwatch assessments of time to perceptible and meaningful pain relief. Baseline pain intensity for each group is shown in Table 9. Median time to onset of analgesia is shown in Table 10.

**Table 9. Baseline pain intensity**

| **Pain Scale** | **Test Composition 2** | **Test Composition 3** | **Test Composition 4** |
|---|---|---|---|
| CPS | (%) | | |
| Moderate | 56 | 56 | 57 |
| Severe | 44 | 44 | 43 |
| VAS | **0 to 100 mm** | | |
| Mean | 73.29 | 72.78 | 73.86 |

These data show that patients in each test group had comparable baseline pain intensity.

**Table 10. Median time to onset of analgesia**

| **Treatment** | **Time (min)** |
|---|---|
| Placebo | >1440 |
| Test Composition 2 | 31 |
| Test Composition 3 | 28 |
| Test Composition 4 | 31 |

As determined in a similar pain study reported in International Patent Publication No. WO 01/91750, 200 mg Celebrex® capsules exhibit a median time to onset of analgesia of 41 minutes. The data in Table 12 show that patients taking Test Compositions 2, 3 or 4 experienced a relatively fast median time to onset of analgesia of 31 minutes or less.

## Claims

1. A process for preparing a finely self-emulsifiable pharmaceutical composition comprising a drug having a solubility in water of not more than 10 mg/ml at 37°C, the process comprising the steps of:
(a) providing at least one pharmaceutically acceptable fatty acid and at least one pharmaceutically acceptable organic amine;
(b) providing a pharmaceutically acceptable solvent for the at least one fatty acid and a pharmaceutically acceptable solvent for the at least one organic amine;
(c) admixing together with the at least one fatty acid the solvent for the at least one fatty acid and the solvent for the at least one organic amine to form a pro-mix in which the fatty acid is substantially completely dissolved;
(d) admixing together with the pre-mix the at least one organic amine to form a mixture in which the organic amine is substantially completely dissolved; and
(e) admixing together with the mixture the drug of low water solubility in dissolved and/or solubilized form to form a pharmaceutical composition.

2. The process of Claim 1 wherein steps (c) and (d) are performed at a temperature of 40°C to 60°C and step (e) is performed at a temperature of 15°C to 30°C.

3. The process of Claim 1 wherein steps (c) and (d) are performed at a temperature of 45°C to 55°C and step (e) is performed at a temperature of 20°C to 25°C.

4. The process of Claim 1 wherein step (c) is performed prior to step (d).

5. The process of Claim 1 wherein steps (c) and (d) are performed simultaneously.

6. The process of Claim 1 wherein the pharmaceutically acceptable solvent for the at least one fatty acid is selected from the group consisting of glycols, alcohols, oleic and linoleic acid triglycerides, caprylic/capric triglycerides, caprylic/capric mono- and diglycerides, polyoxyethylene caprylic/capric glycerides, propylene glycol fatty acid esters, lower alkyl esters of fatty acids, water, and mixtures thereof.

7. The process of Claim 1 wherein the pharmaceutically acceptable solvent for the at least one fatty acid is selected from the group consisting of ethanol, n-butanol, soybean oil, propylene glycol laurate, polyoxyethylene (35) castor oil, polyoxyethylene glyceryl trioleate, ethyl butyrate, ethyl caprylate, ethyl oleate, and mixtures thereof.

8. The process of Claim 1 wherein the pharmaceutically acceptable solvent for the at least one fatty acid is ethanol.

9. The process of Claim 1 wherein the pharmaceutically acceptable solvent for the at least one organic amine is selected from the group consisting of pharmaceutically acceptable glycols, alcohols, oleic and linoleic acid triglycerides, caprylic/capric triglycerides, caprylic/capric mono- and diglycerides, polyoxyethylene caprylic/capric glycerides, propylene glycol fatty acid esters, lower alkyl esters of fatty acids, water and mixtures thereof.

10. The process of Claim 1 wherein the pharmaceutically acceptable solvent for the at least one organic amine is water.

11. The process of Claim 1 wherein the at least one pharmaceutically acceptable organic amine is a tertiary amine.

12. The process of Claim 1 wherein the at least one organic amine is selected from the group consisting of dimethylaminoethanol and triethanolamine.

13. The process of Claim 1 wherein the at least one pharmaceutically acceptable fatty acid has a saturated or unsaturated C₆₋₂₄ carbon chain.

14. The process of Claim 1 wherein the at least one fatty acid is selected from the group consisting of oleic acid, octanoic acid, caproic acid, caprylic acid, capric acid, eleostearic acid, lauric acid, myristic acid, palmitic acid, stearic acid, icosanoic acid, elaidic acid, linoleic acid, linolenic acid, eicosapentaenoic acid and docosahexaenoic acid.

15. The process of Claim 1 wherein the at least one fatty acid is oleic acid.

16. The process of Claim 1 wherein the pharmaceutically acceptable solvent for the at least one fatty acid and the pharmaceutically acceptable solvent for the at least one organic amine are the same.

17. The process of Claim 1 wherein the pharmaceutically acceptable solvent for the at least one fatty acid and the pharmaceutically acceptable solvent for the at least one organic amine are different.

## Patentansprüche

1. Verfahren zum Herstellen einer fein selbstemulgierbaren pharmazeutischen Zusammensetzung, umfassend einen Wirkstoff mit einer Löslichkeit in Wasser bei 37°C von nicht mehr als 10 mg/ml, wobei das Verfahren die folgenden Stufen umfasst:
(a) Bereitstellen mindestens einer pharmazeutisch verträglichen Fettsäure und mindestens eines pharmazeutisch verträglichen organischen Amins;
(b) Bereitstellen eines pharmazeutisch verträglichen Lösungsmittels für die mindestens eine Fettsäure und eines pharmazeutisch verträglichen Lösungsmittels für das mindestens eine organische Amin;
(c) miteinander Mischen des Lösungsmittels für die mindestens eine Fettsäure mit der mindestens einen Fettsäure und des Lösungsmittels für das mindestens eine organische Amin unter Bildung einer Vormischung, in welcher die Fettsäure im Wesentlichen vollständig gelöst ist;
(d) miteinander Mischen des mindestens einen organischen Amins mit der Vormischung unter Bildung eines Gemischs, in dem das organische Amin im Wesentlichen vollständig gelöst ist; und
(e) miteinander Mischen des Wirkstoffs mit geringer Wasserlöslichkeit in gelöster und/oder solubilisierter Form mit dem Gemisch unter Bildung einer pharmazeutischen Zusammensetzung.

2. Verfahren nach Anspruch 1, wobei die Stufen (c) und (d) bei einer Temperatur von 40°C bis 60°C durchgeführt werden und die Stufe (e) bei einer Temperatur von 15°C bis 30°C durchgeführt wird.

3. Verfahren nach Anspruch 1, wobei die Stufen (c) und (d) bei einer Temperatur von 45°C bis 55°C durchgeführt werden und die Stufe (e) bei einer Temperatur von 20°C bis 25°C durchgeführt wird.

4. Verfahren nach Anspruch 1, wobei die Stufe (c) vor der Stufe (d) durchgeführt wird.

5. Verfahren nach Anspruch 1, wobei die Stufen (c) und (d) simultan durchgeführt werden.

6. Verfahren nach Anspruch 1, wobei das pharmazeutisch verträgliche Lösungsmittel für die mindestens eine Fettsäure ausgewählt ist aus der Gruppe, bestehend aus Glykolen, Alkoholen, Öl- und Linolsäuretriglyceriden, Capryl/Caprinsäuretriglyceriden, Capryl/Caprinsäuremono- und -diglyceriden, Polyoxyethylen-Capryl/Caprinsäureglyceriden, Propylenglykolfettsäureestern, Niederalkylestern von Fettsäuren, Wasser und Gemischen davon.

7. Verfahren nach Anspruch 1, wobei das pharmazeutisch verträgliche Lösungsmittel für die mindestens eine Fettsäure ausgewählt ist aus der Gruppe, bestehend aus Ethanol, n-Butanol, Sojabohnenöl, Propylenglykollaurat, Polyoxyethylen (35)-rizinusöl, Polyoxyethylenglyceryltrioleat, Ethylbutyrat, Ethylcaprylat, Ethyloleat und Gemischen davon.

8. Verfahren nach Anspruch 1, wobei das pharmazeutisch verträgliche Lösungsmittel für die mindestens eine Fettsäure Ethanol ist.

9. Verfahren nach Anspruch 1, wobei das pharmazeutisch verträgliche Lösungsmittel für das mindestens eine organische Amin ausgewählt ist aus der Gruppe, bestehend aus pharmazeutisch verträglichen Glykolen, Alkoholen, Öl- und Linolsäuretriglyceriden, Capryl/Caprinsäuretriglyceriden, Capryl/Caprinsäuremono- und -diglyceriden, Polyoxyethylen-Capryl/Caprinsäureglyceriden, Propylenglykolfettsäureestern, Niederalkylestern von Fettsäuren, Wasser und Gemischen davon.

10. Verfahren nach Anspruch 1, wobei das pharmazeutisch verträgliche Lösungsmittel für das mindestens eine organische Amin Wasser ist.

11. Verfahren nach Anspruch 1, wobei das mindestens eine pharmazeutisch verträgliche organische Amin ein tertiäres Amin ist.

12. Verfahren nach Anspruch 1, wobei das mindestens eine organische Amin ausgewählt ist aus der Gruppe, bestehend aus Dimethylaminoethanol und Triethanolamin.

13. Verfahren nach Anspruch 1, wobei die mindestens eine pharmazeutisch verträgliche Fettsäure eine gesättigte oder ungesättigte C₆₋₂₄-Kohlenstoffkette aufweist.

14. Verfahren nach Anspruch 1, wobei die mindestens eine Fettsäure ausgewählt ist aus der Gruppe, bestehend aus Ölsäure, Octansäure, Capronsäure, Caprylsäure, Caprinsäure, Elaeostearinsäure, Laurinsäure, Myristinsäure, Palmitinsäure, Stearinsäure, Eicosansäure, Elaidinsäure, Linolsäure, Linolensäure, Eicosapentaensäure und Docosahexaensäure.

15. Verfahren nach Anspruch 1, wobei die mindestens eine Fettsäure Ölsäure ist.

16. Verfahren nach Anspruch 1, wobei das pharmazeutisch verträgliche Lösungsmittel für die mindestens eine Fettsäure und das pharmazeutisch verträgliche Lösungsmittel für das mindestens eine organische Amin das Gleiche ist.

17. Verfahren nach Anspruch 1, wobei das pharmazeutisch verträgliche Lösungsmittel für die mindestens eine Fettsäure und das pharmazeutisch verträgliche Lösungsmittel für das mindestens eine organische Amin verschieden sind.

## Revendications

1. Procédé de préparation d'une composition pharmaceutique finement auto-émulsifiable, comprenant un médicament ayant une solubilité dans l'eau qui n'est pas supérieure à 10 mg/ml à 37°C, le procédé comprenant les étapes de :
(a) fourniture d'au moins un acide gras pharmaceutiquement acceptable et d'au moins une amine organique pharmaceutiquement acceptable ;
(b) fourniture d'un solvant pharmaceutiquement acceptable pour ledit au moins un acide gras et d'un solvant pharmaceutiquement acceptable pour ladite au moins une amine organique ;
(c) mélangeage dudit au moins un acide gras, du solvant pour ledit au moins un acide gras et du solvant pour ladite au moins une amine organique pour former un prémélange dans lequel l'acide gras est sensiblement totalement dissous ;
(d) mélangeage, avec ledit prémélange, de ladite au moins une amine organique pour former un mélange dans lequel l'amine organique est sensiblement totalement dissoute ; et
(e) mélangeage, avec le mélange, du médicament ayant une faible solubilité dans l'eau, à l'état dissout et/ou solubilisé, pour former une composition pharmaceutique.

2. Procédé selon la revendication 1, dans lequel les étapes (c) et (d) sont mises en oeuvre à une température comprise entre 40°C et 60°C et l'étape (e) est mise en oeuvre à une température comprise entre 15°C et 30°C.

3. Procédé selon la revendication 1, dans lequel les étapes (c) et (d) sont mises en oeuvre à une température comprise entre 45°C et 55°C et l'étape (e) est mise en oeuvre à une température comprise entre 20°C et 25°C.

4. Procédé selon la revendication 1, dans lequel l'étape (c) est mise en oeuvre avant l'étape (d).

5. Procédé selon la revendication 1, dans lequel les étapes (c) et (d) sont mises en oeuvre simultanément.

6. Procédé selon la revendication 1, dans lequel le solvant pharmaceutiquement acceptable pour ledit au moins un acide gras est sélectionné dans le groupe consistant en les glycols, les alcools, les triglycérides des acides oléique et linoléique, les triglycérides capryliques/capriques, les mono- et diglycérides capryliques/capriques, les glycérides capryliques/capriques du polyoxyéthylène, les esters d'acides gras et du propylène glycol, les esters d'alkyle inférieur d'acides gras, l'eau et leurs mélanges.

7. Procédé selon la revendication 1, dans lequel le solvant pharmaceutiquement acceptable pour ledit au moins un acide gras est sélectionné dans le groupe consistant en l'éthanol, le n-butanol, l'huile de soja, le laurate de propylène glycol, l'huile de ricin - polyoxyéthylène (35), le trioléate de glycéryle et de polyoxyéthylène, le butyrate d'éthyle, le caprylate d'éthyle, l'oléate d'éthyle, et leurs mélanges.

8. Procédé selon la revendication 1, dans lequel le solvant pharmaceutiquement acceptable pour ledit au moins un acide gras est l'éthanol.

9. Procédé selon la revendication 1, dans lequel le solvant pharmaceutiquement acceptable pour ladite au moins une amine organique est sélectionné dans le groupe consistant en les glycols pharmaceutiquement acceptables, les alcools, les triglycérides des acides oléique et linoléique, les triglycérides capryliques/capriques, les mono- et diglycérides capryliques/capriques, les glycérides capryliques/capriques du polyoxyéthylène, les esters d'acides gras et du propylène glycol, les esters d'alkyle inférieur d'acides gras, l'eau et leurs mélanges.

10. Procédé selon la revendication 1, dans lequel le solvant pharmaceutiquement acceptable pour ladite au moins une amine organique est l'eau.

11. Procédé selon la revendication 1, dans lequel ladite au moins une amine organique pharmaceutiquement acceptable est une amine tertiaire.

12. Procédé selon la revendication 1, dans lequel ladite au moins une amine organique est sélectionnée dans le groupe consistant en le diméthylaminoéthanol et la triéthanolamine.

13. Procédé selon la revendication 1, dans lequel ledit au moins un acide gras pharmaceutiquement acceptable comporte une chaîne carbonée en C₆₋₂₄ saturée ou insaturée.

14. Procédé selon la revendication 1, dans lequel ledit au moins un acide gras est sélectionné dans le groupe consistant en l'acide oléique, l'acide octanoïque, l'acide caproïque, l'acide caprylique, l'acide caprique, l'acide éléostéarique, l'acide laurique, l'acide myristique, l'acide palmitique, l'acide stéarique, l'acide icosanoïque, l'acide élaïdique, l'acide linoléique, l'acide linolénique, l'acide eicosapentaènoïque et l'acide docosahexaènoïque.

15. Procédé selon la revendication 1, dans lequel ledit au moins un acide gras est l'acide oléique.

16. Procédé selon la revendication 1, dans lequel le solvant pharmaceutiquement acceptable pour ledit au moins un acide gras et le solvant pharmaceutiquement acceptable pour ladite au moins une amine organique sont identiques.

17. Procédé selon la revendication 1, dans lequel le solvant pharmaceutiquement acceptable pour ledit au moins un acide gras et le solvant pharmaceutiquement acceptable pour ladite au moins une amine organique sont différents.
